# EUROPEAN PATENT SPECIFICATION

(11) **EP 4 341 009 B1**
(45) Date of publication and mention of the grant of the patent: **11.11.2026**
(21) Application number: 21844375.2
(22) Date of filing: 15.12.2021
(51) Int. Cl.: B06B 1/02

(54) **AN APPARATUS FOR TRANSMITTING ULTRASONIC WAVES**
VORRICHTUNG ZUM ÜBERTRAGEN VON ULTRASHALLWELLEN
APPAREIL POUR TRANSMETTRE DES ONDES ULTRASONORES

(43) Date of publication of application: 27.03.2024
(60) Divisional application: 26202681.8 / 4 825 500
(73) Proprietor: Shaheen Innovations Holding Limited, Abu Dhabi (AE)
(72) Inventor: ALSHAIBA SALEH GHANNAM ALMAZROUEI, Mohammed, Abu Dhabi (AE); MACHOVEC, Jeff, Abu Dhabi (AE); LAMOUREUX, Clement, Abu Dhabi (AE)
(74) Representative: Forresters IP LLP
(86) International application number: PCT/GB2021/053310
(87) International publication number: WO 2023/111496

(56) References cited:
- EP-A1- 3 505 098
- WO-A2-2012/138835
- US-A- 6 011 345
- US-A1- 2019 056 131

## Description

### FIELD

The present invention relates to an apparatus for transmitting ultrasonic waves. The present invention more particularly relates an apparatus for transmitting ultrasonic waves which comprises a microchip which drives a resonant circuit in the form of an LC tank, an antenna or a piezoelectric transducer.

### BACKGROUND

Therapeutic aerosol delivery is the mainstay for the treatment of asthma, chronic obstructive pulmonary disease (COPD) and cystic fibrosis. Therapeutic aerosol also has applications for the treatment of influenza, osteoporosis as well as the delivery of vaccines.

Pulmonary delivery of therapeutics for the treatment of non-respiratory systemic disease is appealing because of high lung vascularity, a thin blood-alveolar barrier, large surface area, avoidance of gastric enzymes and first-pass hepatic metabolism. It is also appealing because of improved patient comfort and adherence. The pulmonary system can be leveraged to deliver antibodies, proteins, pain killers and nucleic acids. The treatment of central nervous system disorders, such as tobacco dependence, could be significantly enhanced through the efficient delivery of nicotine to the systematic circulation through the lungs.

The effectiveness of therapeutic aerosol relates to the amount of drug deposited beyond the oropharyngeal region. The region where the deposit occurs is a function of the inhaled particles size.

The devices currently used for the administration of inhaled drugs are divided into three categories: nebulizers, metered-dose inhalers, and dry powder inhalers. Nebulizers are typically divided into two types: jet and ultrasonic but in conventional devices both types have weaknesses and present issues.

Jet nebulizers are based on the Bernoulli principle and produce relatively large droplets that generally deposit in the oropharyngeal region and are therefore not particularly effective. Ultrasonic nebulizers use piezoelectric crystals that vibrate at frequencies, ranging between 1 MHz and 1.7 MHz, transmitting the vibratory energy to the liquid converting it to aerosol. It is acknowledged that ultrasonic nebulizers are not effective if viscous suspensions or solutions are used and tend to heat the medication, hence destroy the molecules and remove the benefits of inhalation.

There are other applications which require a resonant circuit to be driven efficiently in an optimal manner at or near the resonant frequency of the resonant circuit. For instance, a device which incorporates a resonant circuit in the form of an antenna typically needs to drive the antenna with a precise AC drive signal to enable the antenna to function optimally. In addition, devices which incorporate a resonant circuit in the form an ultrasonic piezoelectric transducer must produce an AC drive signal to drive the ultrasonic transducer optimally.

Thus, a need exists in the art for an apparatus for transmitting ultrasonic waves which seeks to address at least some of the problems described herein.

EP 3 505 098 A1 discloses various systems and methods for controlling the activation of energy surgical instruments.

### BRIEF DESCRIPTION OF THE INVENTION

The present invention provides an apparatus as claimed in claim 1. The present invention also provides preferred embodiments as claimed in the dependent claims.

The various examples of this disclosure which are described below have multiple benefits and advantages over conventional apparatuses. These benefits and advantages are set out in the description below.

Since the apparatuses of examples of this disclosure enable higher efficiency operation than conventional apparatuses, the apparatuses of examples of this disclosure have an environmental benefit due to the reduced power requirement.

### BRIEF DESCRIPTION OF THE FIGURES

In order that the present disclosure may be more readily understood, preferable embodiments thereof will now be described, by way of example only, with reference to the accompanying drawings, in which:
Figure 1 is a schematic diagram of an apparatus of this disclosure;
Figure 2 is a schematic diagram of an integrated circuit of this disclosure;
Figure 3 is a schematic diagram of a pulse width modulation generator of this disclosure;
Figure 4 is timing diagram of an example of this disclosure;
Figure 5 is timing diagram of an example of this disclosure;
Figure 6 is a table showing port functions of an example of this disclosure;
Figure 7 is a schematic diagram of an integrated circuit of this disclosure;
Figure 8 is a circuit diagram of an H-bridge of an example of this disclosure;
Figure 9 is a circuit diagram of a current sense arrangement of an example of this disclosure;
Figure 10 is a circuit diagram of an H-bridge of an example of this disclosure;
Figure 11 is a graph showing the voltages during the phases of operation of the H-bridge of figure 8;
Figure 12 is a graph showing the voltages during the phases of operation of the H-bridge of Figure 8;
Figure 13 is a graph showing the voltage and current at a terminal of an ultrasonic transducer while the ultrasonic transducer is being driven by the H-bridge of Figure 8;
Figure 14 is a schematic diagram showing connections between integrated circuits of this disclosure;
Figure 15 is a schematic diagram of an integrated circuit of this disclosure; and
Figure 16 is diagram illustrating the steps of an authentication method of an example of this disclosure.

### DETAILED DESCRIPTION OF THE DISCLOSURE

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion.

The following disclosure provides many different embodiments, or examples, for implementing different features of the provided subject matter. Specific examples of components, concentrations, applications and arrangements are described below to simplify the present disclosure. These are, of course, merely examples and are not intended to be limiting. For example, the attachment of a first feature and a second feature in the description that follows may include embodiments in which the first feature and the second feature are attached in direct contact, and may also include embodiments in which additional features may be positioned between the first feature and the second feature, such that the first feature and the second feature may not be in direct contact. In addition, the present disclosure may repeat reference numerals and/or letters in the various examples. This repetition is for the purpose of simplicity and clarity and does not in itself dictate a relationship between the various embodiments and/or configurations discussed.

The following disclosure describes representative examples. Each example may be considered to be an embodiment and any reference to an "example" may be changed to "embodiment" in the present disclosure.

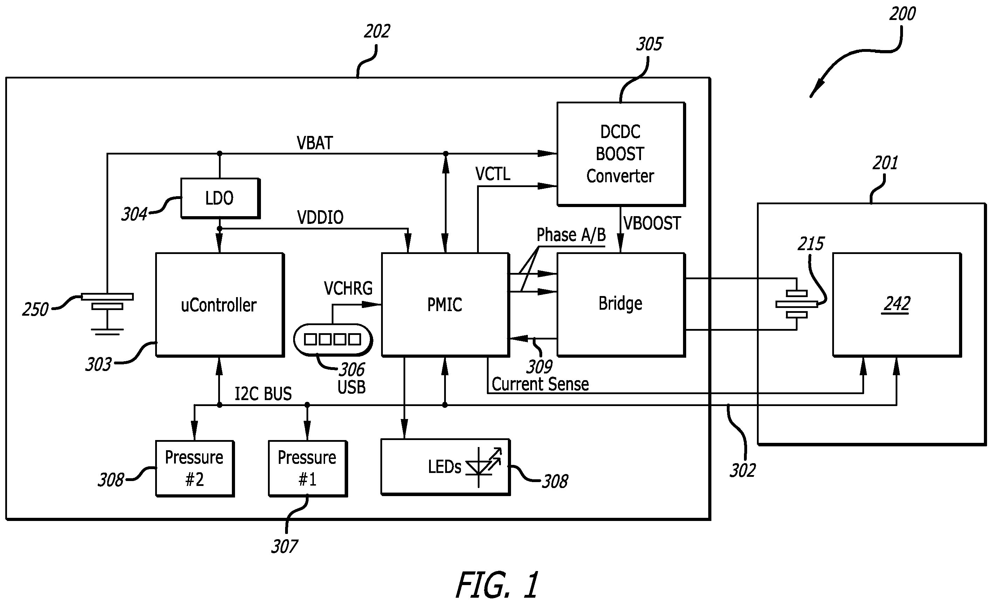

Referring now to Figure 1 of the accompanying drawings, a driver device 202 comprises a microchip which is referred to herein as a power management integrated circuit or PMIC 300. The PMIC 300 is a microchip for driving a resonant circuit. The resonant circuit is an LC tank, an antenna or a piezoelectric transducer. In this example, the resonant circuit is an ultrasonic transducer 215 which is provided within a resonant circuit device 201. In this example, the resonant circuit device 201 is a separate device which is releasably coupled to the driver device 202. In other examples, the elements of the resonant circuit device 201 are combined in the same device as the driver device 202.

In this disclosure, the terms chip, microchip and integrated circuit are interchangeable. The microchip or integrated circuit is a single unit which comprises a plurality of interconnected embedded components and subsystems. The microchip is, for example, at least partly of a semiconductor, such as silicon, and is fabricated using semiconductor manufacturing techniques.

This disclosure describes an example resonant circuit device 201 which is a mist inhaler device which comprises an ultrasonic transducer 215. When the ultrasonic transducer 215 is activated by the driver device 202, the ultrasonic transducer 215 atomises a liquid to generate a mist for inhalation by a user.

However, it is to be appreciated that the elements of the driver device are used differently in other applications involving a resonant circuit. In these other examples, the ultrasonic transducer 215 is replaced with another resonant circuit, such as LC tank or an antenna.

In one example, the resonant circuit device 201 comprises a resonant circuit in the form of a piezoelectric ultrasonic transducer which generates ultrasonic waves that are used for wireless power transfer. In this example, the ultrasonic transducer is driven by the driver device 202 to generate ultrasonic waves that can be focused and sent to a receiver transducer. The receiver transducer converts the ultrasonic waves back into electrical energy and stores the energy in an energy storage device, such as a battery, or uses the electrical energy to power a device. In this way, a device can be remotely charged or powered via the power which is transferred wirelessly via the ultrasonic waves without the device having to be tethered to an electrical outlet.

As described below, the driver device 202 is configured to modulate the frequency and duty cycle of the AC power signal driving an ultrasonic transducer with high accuracy and efficiency. In the case of a power transfer system, this enables the driver device 202 to encode information for wireless transmission by modulating the ultrasonic waves which carry the encoded information. In one example, the driver device 202 is configured for use in a wireless power transfer system such as but not limited to the type described in US patent no. 9,001,622 entitled "receiver communications for wireless power transfer".

In another example, the driver device 202 drives a resonant circuit in the form of an ultrasonic transducer for delivering ultrasonic waves at a precise frequency and at high intensity to treat tumours. The high-intensity focused ultrasound from the ultrasonic transducer is used as a non-invasive targeted treatment to raise the temperature within a tumour to above 65°C, killing the cells of the tumour without damaging the surrounding tissue.

In a further application, the driver device 202 is used to drive a resonant circuit in the form an antenna to control the frequency and power of waves transmitted by the antenna accurately. In this example, the antenna may be used for any purpose. In one example, the driver device 202 drives an antenna to transmit waves at a precise frequency for the purpose of searching for materials, such as minerals or gold in the ground.

The driver device 202 comprises a second microchip which is referred to herein as a bridge integrated circuit or bridge IC 301 which is electrically connected to the PMIC 300. The bridge IC 301 is a microchip for driving a resonant circuit, such as an LC tank, an antenna or a piezoelectric transducer. The bridge IC 301 is a single unit which comprises a plurality of interconnected embedded components and subsystems.

In this example, the PMIC 300 and the bridge IC 301 are mounted to the same PCB of the driver device 202. In this example, the physical dimensions of the PMIC 300 are 1-3mm wide and 1-3mm long and the physical dimensions of the bridge IC 301 are 1-3mm wide and 1-3mm long.

In this example, the resonant circuit device 201 comprises an optional programmable or one time programmable integrated circuit or OTP IC 242. When the resonant circuit device 201 is coupled to the driver device 202, the OTP IC is electrically connected to the PMIC 300 to receive power from the PMIC 300 such that the PMIC 300 can manage the voltage supplied to the OTP IC 242. The OTP IC 242 is also connected to a communication bus 302 in the driver device 202. In this example, the communication bus 302 is an I2C bus but in other examples the communication bus 302 is another type of digital serial communication bus.

The OTP IC 242 provides a security function which is described below. However, it is to be appreciated that the OTP IC 242 is omitted in examples of this disclosure which do not require such a security function.

The ultrasonic transducer 215 in the resonant circuit device 201 is electrically connected to the bridge IC 301 so that the ultrasonic transducer 215 may be driven by an AC drive signal generated by the bridge IC 301 when the device 200 is in use.

The driver device 202 comprises a processor in the form of a microcontroller 303 which is electrically coupled for communication with the communication bus 302. In this example, the microcontroller 303 is a Bluetooth^{™} low energy (BLE) microcontroller but in other examples the microcontroller 303 is a general purpose processor. The microcontroller 303 receives power from a low dropout regulator (LDO) 304 which is driven by the battery 250. The LDO 304 provides a stable regulated voltage to the microcontroller 303 to enable the microcontroller 303 to operate consistently even when there is a variation in the voltage of the battery 250.

The driver device 202 comprises a voltage regulator in the form a DC-DC boost converter 305 which is powered by the battery 250. The boost converter 305 increases the voltage of the battery 250 to a programmable voltage VBOOST. The programmable voltage VBOOST is set by the boost converter 305 in response to a voltage control signal VCTL from the PMIC 300. As will be described in more detail below, the boost converter 305 outputs the voltage VBOOST to the bridge IC 301. In other examples, the voltage regulator is a buck converter or another type of voltage regulator which outputs a selectable voltage.

The voltage control signal VCTL is generated by a digital to analogue converter (DAC) which, in this example, is implemented within the PMIC 300. The DAC is not visible in Figure 1 since the DAC is integrated within the PMIC 300. The DAC and the technical benefits of integrating the DAC within the PMIC 300 are described in detail below.

In this example, the PMIC 300 is connected to a power source connector in the form of a universal serial bus (USB) connector 306 so that the PMIC 300 can receive a charging voltage VCHRG when the USB connector 306 is coupled to a USB charger.

In this example, the driver device 202 comprises a first pressure sensor 307 which, in this example, is a static pressure sensor. The driver device 202 also comprises a second pressure sensor 308 which, in this example, is a dynamic pressure sensor. However, in other examples, the driver device 202 comprises only one of the two pressure sensors 307, 308 or the pressure sensors 307, 308 are omitted entirely. In this example, which relates to a mist generator device, the pressure sensors 307, 308 sense a change in the pressure in an aerosol chamber (not shown) to sense when a user is drawing mist from the aerosol chamber.

In this example, the driver device 202 comprises a plurality of LEDs 308 which are controlled by the PMIC 300. In other examples, the LEDs 308 are omitted.

The microcontroller 303 functions as a master device on the communication bus 302, with the PMIC 300 being a first slave device, the OTP IC 242 being a second slave device, the second pressure sensor 308 being a third slave device and the first pressure sensor 307 being the a fourth slave device. The communication bus 302 enables the microcontroller 303 to control the following functions within the driver device 202:
1. All functions of the PMIC are highly configurable by the microcontroller 303.
2. The current flowing through the resonant circuit (ultrasonic transducer 215) is sensed by a high bandwidth sense and rectifier circuit at a high common mode voltage (high side of the bridge). The sensed current is converted into a voltage proportional to the rms current and provided as a buffered voltage at a current sense output pin 309 of the bridge IC 301. This voltage is fed to and sampled in the PMIC 300 and made available as a digital representation via I2C requests. Sensing the current flowing through the ultrasonic transducer 215 forms part of the resonant frequency tracking functionality. As described herein, the ability of the device to enable this functionality within the bridge IC 301 provides significant technical benefits.
3. The DAC (not shown in Figure 1) integrated within the PMIC 300 enables the DC-DC boost converter voltage VBOOST to be programmed to be between 10V and 20V.
4. The microcontroller 303 enables the charger sub-system of the device 202 to manage the charging of a battery, which in this example is a single cell battery.
5. A Light Emitting Diode (LED) driver module (not shown) is powered by the PMIC 300 to drive and dim digitally the LEDs 308 either in linear mode or in gamma corrected mode.
6. The microcontroller 303 is able to read Pressure#1 and Pressure#2 sensor values from the pressure sensors 307, 308.

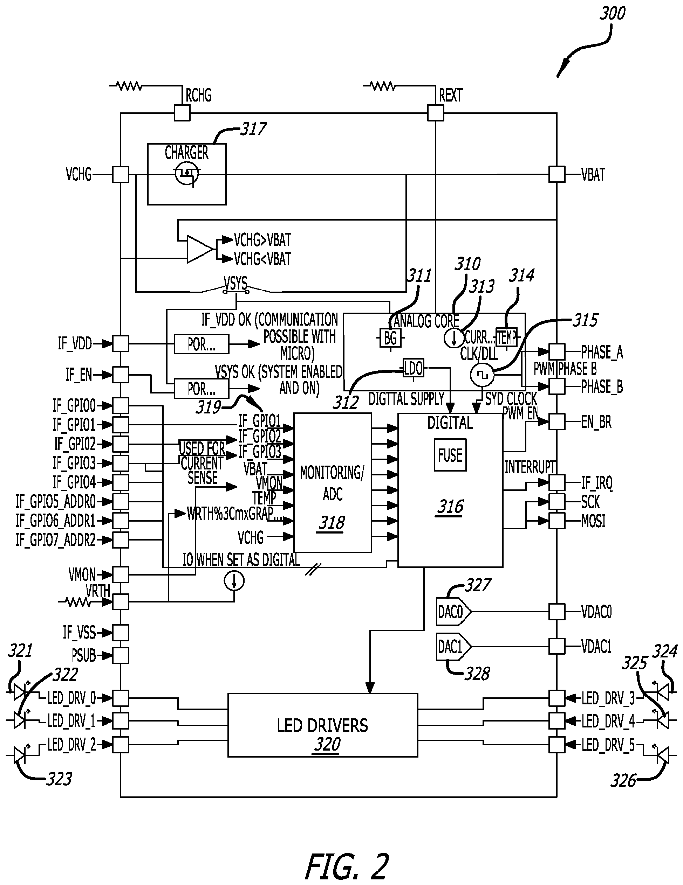

Referring now to Figure 2 of the accompanying drawings, the PMIC 300 is, in this example, a self-contained chip or integrated circuit which comprises integrated subsystems and a plurality of pins which provide electrical inputs and outputs to the PMIC 300. The references to an integrated circuit or chip in this disclosure are interchangeable and either term encompasses a semiconductor device which may, for instance, be of silicon.

The PMIC 300 comprises an analogue core 310 which comprises analogue components including a reference block (BG) 311, a LDO 312, a current sensor 313, a temperature sensor 314 and an oscillator 315.

As described in more detail below, the oscillator 315 is coupled to a delay locked loop (DLL) which outputs pulse width modulation (PWM) phases A and B. The oscillator 315 and the DLL generate a two phase centre aligned PWM output which drives an H bridge in the bridge IC 301.

The DLL comprises a plurality of delay lines connected end to end, wherein the total delay of the delay lines is equal to the period of the main clock signal clk_m. In this example, the DLL is implemented in a digital processor subsystem, referred to herein as a digital core 316, of the PMIC 300 which receives a clock signal from the oscillator 315 and a regulated power supply voltage from the LDO 312. The DLL is implemented in a large number (e.g. in the order of millions) of delay gates which are connected end to end in the digital core 316.

The implementation of the oscillator 315 and the DLL in the same integrated circuit of the PMIC 300 in order to generate a two phase centre aligned PWM signal is unique since at present no signal generator component in the integrated circuit market comprises this implementation.

As described herein, PWM is part of the functionality which enables the driver device 202 to track the resonant frequency of the ultrasonic transducer 215 accurately in order to maintain an efficient transfer from electrical energy to kinetic energy in order to optimise the generation of mist. The same functionality enables other examples which comprise a different resonant circuit by driving the resonant circuit efficiently and at a high power and a high frequency.

In this example, the PMIC 300 comprises a charger circuit 317 which controls the charging of the battery 250, for instance by power from a USB power source. The charger circuit 317 is omitted in other examples which do not require a battery.

The PMIC 300 comprises an integrated power switch VSYS which configures the PMIC 300 to power the analogue core 310 by power from the battery 250 or by power from an external power source if the battery 250 is being charged.

The PMIC 300 comprises an embedded analogue to digital converter (ADC) subsystem 318. The implementation of the ADC 318 together with the oscillator 315 in the same integrated circuit is, in itself, unique since there is no other integrated circuit in the integrated circuit market which comprises an oscillator and an ADC implemented as sub-blocks within the integrated circuit. In a conventional device, an ADC is typically provided as a separate discrete component from an oscillator with the separate ADC and oscillator being mounted to the same PCB. The problem with this conventional arrangement is that the two separate components of the ADC and the oscillator take up space unnecessarily on the PCB. A further problem is that the conventional ADC and oscillator are usually connected to one another by a serial data communication bus, such as an I2C bus, which has a limited communication speed of up to only 400 kHz. In contrast to conventional devices, the PMIC 300 comprises the ADC 318 and the oscillator 315 integrated within the same integrated circuit which eliminates any lag in communication between the ADC 318 and the oscillator 315, meaning that the ADC 318 and the oscillator 315 can communicate with one another at high speed, such as at the speed of the oscillator 315 (e.g. 3 MHz to 5 MHz).

In the PMIC 300 of this example, the oscillator 315 is running at 5 MHz and generates a clock signal SYS CLOCK at 5 MHz. However, in other examples, the oscillator 315 generates a clock signal at a much higher frequency of up to 105 MHz. The integrated circuits described herein are all configured to operate at the high frequency of the oscillator 315.

The ADC 318 comprises a plurality of feedback input terminals or analogue inputs 319 which comprise a plurality of GPIO inputs (IF_GPIO1-3). At least one of the feedback input terminals or the analogue inputs 319 receives a feedback signal from an H-bridge circuit in the bridge IC 301, the feedback signal being indicative of a parameter of the operation of the H-bridge circuit or an AC drive signal when the H-bridge circuit is driving a resonant circuit, such as the ultrasonic transducer 215, with the AC drive signal. As described below, the GPIO inputs are used to receive a current sense signal from the bridge IC 301 which is indicative of the route mean square (rms) current reported by the bridge IC 301. In this example, one of the GPIO inputs is a feedback input terminal which receives a feedback signal from the H-bridge in the bridge IC 301.

The ADC subsystem 318 samples analogue signals received at the plurality of ADC input terminals 319 at a sampling frequency which is proportional to the frequency of the main clock signal. The ADC subsystem 318 then generates ADC digital signals using the sampled analogue signals.

In this example, the ADC 318 which is incorporated in the PMIC 300 samples not only the RMS current flowing through the H-bridge 334 and the ultrasonic transducer 215 but also voltages available in the system (e.g. VBAT, VCHRG, VBOOST), the temperature of the PMIC 300, the temperature of the battery 250 and the GPIO inputs (IF_GPIO1-3) which allow for future extensions.

The digital core 316 receives the ADC generated digital signals from the ADC subsystem and processes the ADC digital signals to generate the driver control signal. The digital core 316 communicates the driver control signal to the PWM signal generator subsystem (DLL 332) to control the PWM signal generator subsystem.

Rectification circuits existing in the market today have a very limited bandwidth (typically less than 1 MHz). Since the oscillator 315 of the PMIC 300 is running at up to 5 MHz or even up to 105 Mhz, a high bandwidth rectifier circuit is implemented in the PMIC 300. As will be described below, sensing the RMS current within an H bridge of the bridge IC 301 forms part of a feedback loop which enables the driver device 202 to drive the ultrasonic transducer 215 with high precision. The feedback loop is a game changer in the industry of driving ultrasound transducers since it accommodates for any process variation in the piezo electric transducer production (variations of resonance frequencies) and it compensates for temperature effects of the resonance frequency. This is achieved, in part, by the inventive realisation of integrating the ADC 318, the oscillator 315 and the DLL within the same integrated circuit of the PMIC 300. The integration enables these sub-systems to communicate with one another at high speed (e.g. at the clock frequency of 5 MHz or up to 105 MHz). Reducing the lag between these subsystems is a game changer in the ultrasonics industry, particularly in the field of mist generator devices.

The ADC 318 comprises a battery voltage monitoring input VBAT and a charger input voltage monitoring input VCHG as well as voltage monitoring inputs VMON and VRTH as well as a temperature monitoring input TEMP.

The temperature monitoring input TEMP receives a temperature signal from the temperature sensor 314 which is embedded within the PMIC 300. This enables the PMIC 300 to sense the actual temperature within the PMIC 300 accurately so that the PMIC 300 can detect any malfunction within the PMIC 300 as well as malfunction to other components on the printed circuit board which affect the temperature of the PMIC 300. The PMIC 300 can then control the bridge IC 301 to prevent excitation of the ultrasonic transducer 215 if there is a malfunction in order to maintain the safety of the resonant circuit device 201.

The additional temperature sensor input VRTH receives a temperature sensing signal from an external temperature sensor within the driver device 202 which monitors the temperature of a battery. The PMIC 300 can thus react to stop the battery from being charged in the event of a high battery temperature or otherwise shut down the driver device 202 in order to reduce the risk of damage being caused by an excessively high battery temperature.

The PMIC 300 comprises an LED driver 320 which, in this example, receives a digital drive signal from the digital core 316 and provides LED drive output signals to six LEDs 321-326 which are configured to be coupled to output pins of the PMIC 300. The LED driver 320 can thus drive and dim the LEDs 321-326 in up to six independent channels.

The PMIC 300 comprises a first digital to analogue converter (DAC) 327 which converts digital signals within the PMIC 300 into an analogue voltage control signal which is output from the PMIC 300 via an output pin VDAC0. The first DAC 327 converts a digital control signal generated by the digital core 316 into an analogue voltage control signal which is output via the output pin VDAC0 to control a voltage regulator circuit, such as the boost converter 305. The voltage control signal thus controls the voltage regulator circuit to generate a predetermined voltage for modulation by the H-bridge circuit to drive a resonant circuit, such as the ultrasonic transducer 215, in response to feedback signals which are indicative of the operation of the resonant circuit (the ultrasonic transducer 215).

In this example, the PMIC 300 comprises a second DAC 328 which converts digital signals within the PMIC 300 into an analogue signal which is output from the PMIC 300 via a second analogue output pin VDAC1.

Embedding the DAC 327 or the DACs 327, 328 within the same microchip as the other subsystems of the PMIC 300 allows the DACs 327, 328 to communicate with the digital core 316 and other components within the PMIC 300 at high speed with no or minimal communication lag. The DACs 327, 328 provide analogue outputs which control external feedback loops. For instance, the first DAC 327 provides the control signal VCTL to the boost converter 305 to control the operation of the boost converter 305. In other examples, the DACs 327, 328 are configured to provide a drive signal to a DC-DC buck converter instead of or in addition to the boost converter 305. Integrating the two independent DAC channels in the PMIC 300 enables the PMIC 300 to manipulate the feedback loop of any regulator used in the driver device 202 and allows the driver device 202 to regulate the sonication power of the ultrasonic transducer 215 or to set analogue thresholds for absolute maximum current and temperature settings of the ultrasonic transducer 215.

The PMIC 300 comprises a serial communication interface which, in this example, is an I2C interface which incorporates external I2C address set through pins.

The PMIC 300 also comprises various functional blocks which include a digital machine (FSM) to implement the functionality of the microchip. These blocks will be described in more detail below.

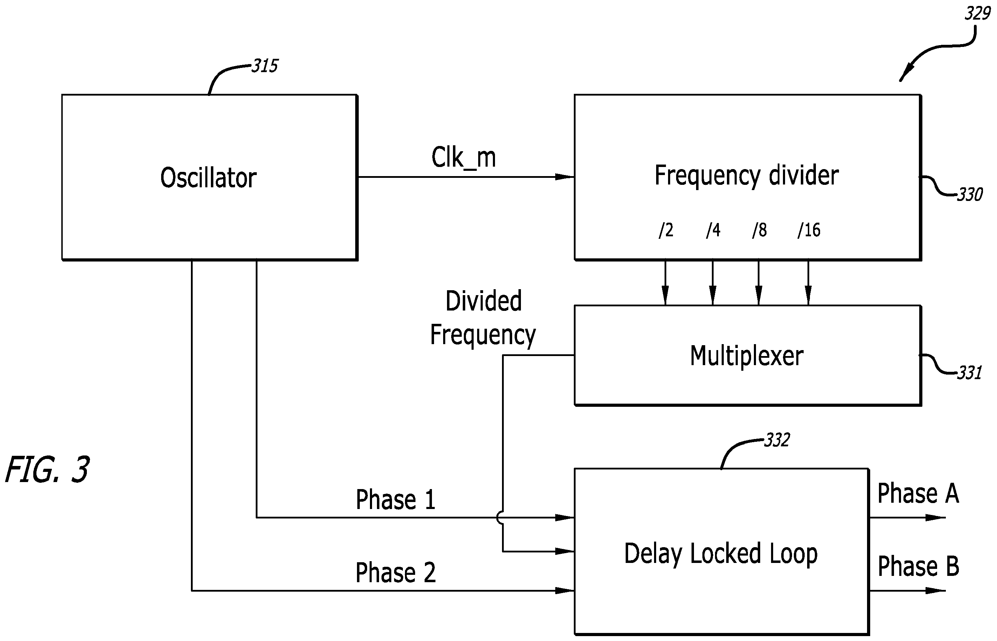

Referring now to Figure 3 of the accompanying drawings, a pulse width modulation (PWM) signal generator subsystem 329 is embedded within the PMIC 300. The PWM generator system 329 comprises the oscillator 315, and frequency divider 330, a multiplexer 331 and a delay locked loop (DLL) 332. As will be described below, the PWM generator system 329 is a two phase centre aligned PWM generator.

The frequency divider 330, the multiplexer 331 and the DLL 332 are implemented in digital logic components (e.g. transistors, logic gates, etc.) within the digital core 316.

In examples of this disclosure, the frequency range which is covered by the oscillator 315 and respectively by the PWM generator system 329 is 50 kHz to 5 MHz or up to 105 MHz. The frequency accuracy of the PWM generator system 329 is ±1% and the spread over temperature is ±1%. In the IC market today, no IC has an embedded oscillator and two phase centre aligned PWM generator that can provide a frequency range of 50 kHz to 5 MHz or up to 105 MHz.

The oscillator 315 generates a main clock signal (clk_m) with a frequency of 50 kHz to 5 MHz or up to 105 MHz. The main clock clk_m is input to the frequency divider 330 which divides the frequency of the main clock clk_m by one or more predetermined divisor amounts. In this example, the frequency divider 330 divides the frequency of the main clock clk_m by 2, 4, 8 and 16 and provides the divided frequency clocks as outputs to the multiplexer 331. The multiplexer 331 multiplexes the divided frequency clocks and provides a divided frequency output to the DLL 332. This signal which is passed to the DLL 332 is a frequency reference signal which controls the **DLL** 332 to output signals at a desired frequency. In other examples, the frequency divider 330 and the multiplexer 331 are omitted.

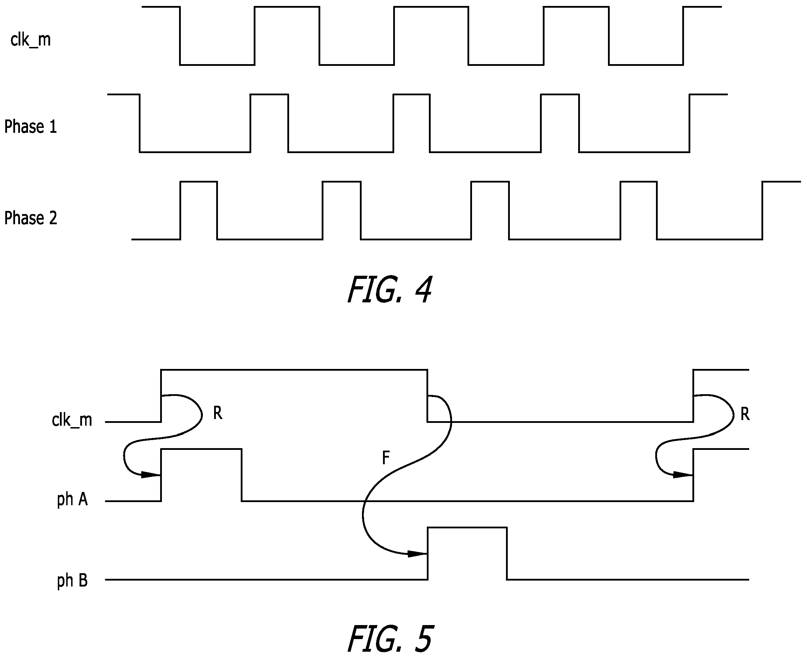

The oscillator 315 also generates two phases; a first phase clock signal Phase 1 and a second phase clock signal Phase 2. The phases of the first phase clock signal and the second phase clock signal are centre aligned. As illustrated in Figure 4:
- The first phase clock signal Phase 1 is high for a variable time of clk_m's positive half-period and low during clk_m's negative half-period.
- The second phase clock signal Phase 2 is high for a variable time of clk_m's negative half-period and low during clk_m's positive half-period.

Phase 1 and Phase 2 are then sent to the **DLL** 332 which generates a double frequency clock signal using the first phase clock signal Phase 1 and the second phase clock signal Phase 2. The double frequency clock signal is double the frequency of the main clock signal clk_m. In this example, an "OR" gate within the **DLL** 332 generates the double frequency clock signal using the first phase clock signal Phase 1 and the second phase clock signal Phase 2. This double frequency clock or the divided frequency coming from the frequency divider 330 is selected based on a target frequency selected and then used as reference for the **DLL** 332.

Within the **DLL** 332, a signal referred to hereafter as "clock" represents the main clock clk_m multiplied by 2, while a signal referred to hereafter as "clock_del" is a replica of clock delayed by one period of the frequency. Clock and clock_del are passed through a phase frequency detector. A node Vc is then charged or discharged by a charge-pump based on the phase error polarity. A control voltage is fed directly to control the delay of every single delay unit within the **DLL** 332 until the total delay of the **DLL** 332 is exactly one period.

The DLL 332 controls the rising edge of the first phase clock signal Phase 1 and the second phase clock signal Phase 2 to be synchronous with the rising edge of the double frequency clock signal. The DLL 332 adjusts the frequency and the duty cycle of the first phase clock signal Phase 1 and the second phase clock signal Phase 2 in response to a respective frequency reference signal and a duty cycle control signal to produce a first phase output signal Phase A and a second phase output signal Phase B to drive an H-bridge or an inverter to generate an AC drive signal to drive an ultrasonic transducer.

The PMIC 300 comprises a first phase output signal terminal PHASE_A which outputs the first phase output signal Phase A to an H-bridge circuit and a second phase output signal terminal PHASE_B which outputs the second phase output signal Phase B to an H-bridge circuit.

In this example, the DLL 332 adjusts the duty cycle of the first phase clock signal Phase 1 and the second phase clock signal Phase 2 in response to the duty cycle control signal by varying the delay of each delay line in the DLL 332 response to the duty cycle control signal.

The clock is used at double of its frequency because guarantees better accuracy. As shown in Figure 5, for the purpose of explanation if the frequency of the main clock clk_m is used (which it is not in examples of this disclosure), Phase A is synchronous with clock's rising edge R, while Phase B is synchronous with clock's falling edge F. The delay line of the DLL 332 controls the rising edge R and so, for the falling edge F, the PWM generator system 329 would need to rely on a perfect matching of the delay units of the DLL 332 which can be imperfect. However, to remove this error, the PWM generator system 329 uses the double frequency clock so that both Phase A and Phase B are synchronous with the rising edge R of the double frequency clock.

To perform a duty-cycle from 20% to 50% with a 2% step size, the delay line of the DLL 332 comprises 25 delay units, with the output of each respective delay unit representing a Phase nth. Eventually the phase of the output of the final delay unit will correspond to the input clock. Considering that all delays will be almost the same, a particular duty cycle is obtained with the output of the specific delay unit with simple logic in the digital core 316.

It is important to take care of the DLL 332 startup as the DLL 332 might not be able to lock a period of delay but two or more periods, taking the DLL 332 to a non-convergence zone. To avoid this issue, a start-up circuit is implemented in the PWM generator system 329 which allows the DLL 332 to start from a known and deterministic condition. The start-up circuit furthermore allows the DLL 332 to start with the minimum delay.

In examples of this disclosure, the frequency range covered by the PWM generator system 329 is extended and so the delay units in the DLL 332 can provide delays of 4 ns (for an oscillator frequency of 5 MHz) to 400 ns (for an oscillator frequency of 50 kHz). In order to accommodate for these differing delays, capacitors Cb are included in the PWM generator system 329, with the capacitor value being selected to provide the required delay.

The Phase A and Phase B are output from the DLL 332 and passed through a digital IO to the bridge IC 301 so that the Phase A and Phase B can be used to control the operation of the bridge IC 301.

The battery charging functionality of the driver device 202 will now be described in more detail. The battery charging sub-system comprises the charger circuit 317 which is embedded in the PMIC 300 and controlled by a digital charge controller hosted in the PMIC 300. The charger circuit 317 is controlled by the microcontroller 303 via the communication bus 302. The battery charging sub-system is able to charge a single cell lithium polymer (LiPo) or lithium-ion (Li-ion) battery.

In this example, the battery charging sub-system is able to charge a battery or batteries with a charging current of up to 1A from a 5V power supply (e.g. a USB power supply). One or more of the following parameters can be programmed through the communication bus 302 (12C interface) to adapt the charge parameters for the battery:
- Charge voltage can be set between 3.9V and 4.3V in 100mV steps.
- The charge current can be set between 150 mA and 1000mA in 50mA steps.
- The pre-charge current is 1/10 of the charge current.
- Pre-charge and fast charge timeouts can be set between 5 and 85 min respectively 20 and 340 min.
- Optionally an external negative temperature coefficient (NTC) thermistor can be used to monitor the battery temperature.

In some examples, the battery charging sub-system reports one or more of the following events by raising an interrupt to the host microcontroller 303:
- Battery detected
- Battery is being charged
- Battery is fully charged
- Battery is not present
- Charge timeout reached
- Charging supply is below the undervoltage limit

The main advantage of having the charger circuit 317 embedded in the PMIC 300, is that it allows all the programming options and event indications listed to be implemented within the PMIC 300 which guarantees the safe operation of the battery charging sub-system. Furthermore, a significant manufacturing cost and PCB space saving can be accomplished compared with conventional resonant circuit devices, such as mist inhaler devices, which comprise discrete components of a charging system mounted separately on a PCB. The charger circuit 317 also allows for highly versatile setting of charge current and voltage, different fault timeouts and numerous event flags for detailed status analysis.

The analogue to digital converter (ADC) 318 will now be described in more detail. The inventors had to overcome significant technical challenges to integrate the ADC 318 within the PMIC 300 with the high speed oscillator 315. Moreover, integrating the ADC 318 within the PMIC 300 goes against the conventional approach in the art which relies on using one of the many discrete ADC devices that are available in the IC market.

In this example, the ADC 318 samples at least one parameter within the ultrasonic transducer driver chip (PMIC 300) at a sampling rate which is equal to the frequency of the main clock signal clk_m. In this example, the ADC 318 is a 10 bit analogue to digital converter which is able to unload digital sampling from the microprocessor 303 to save the resources of the microprocessor 303. Integrating the ADC 318 within the PMIC 300 also avoids the need to use an I2C bus that would otherwise slow down the sampling ability of the ADC (a conventional device relies on an I2C bus to communicate data between a dedicated discrete ADC and a microcontroller at a limited clock speed of typically up to 400 kHz).

In examples of this disclosure, one or more of the following parameters can be sampled sequentially by the ADC 318:
i. An rms current signal which is received at the ultrasonic transducer driver chip (PMIC 300) from an external inverter circuit which is driving an ultrasonic transducer. In this is example, this parameter is a root mean square (rms) current reported by the bridge IC 301. Sensing the rms current is important to implementing the feedback loop used for driving the ultrasound transducer 215. The ADC 318 is able to sense the rms current directly from the bridge IC 301 via a signal with minimal or no lag since the ADC 318 does not rely on this information being transmitted via an I2C bus. This provides a significant speed and accuracy benefit over conventional devices which are constrained by the comparatively low speeds of an I2C bus.
ii. The voltage of a battery connected to the PMIC 300.
iii. The voltage of a charger connected to the PMIC 300.
iv. A temperature signal, such as a temperature signal which is indicative of the PMIC 300 chip temperature. As described above, this temperature can be measured very accurately due to the temperature sensor 314 being embedded in the same IC as the oscillator 315. For example, if the PMIC 300 temperature goes up, the current, frequency and PWM are regulated by the PMIC 300 to control the transducer oscillation which in turn controls the temperature.
v. Two external pins.
vi. External NTC temperature sensor to monitor battery pack temperature.

In some examples, the ADC 318 samples one or more of the above-mentioned sources sequentially, for instance in a round robin scheme. The ADC 318 samples the sources at high speed, such as the speed of the oscillator 315 which may be up to 5 MHz or up to 105 MHz.

In some examples, the device 202 is configured so that a user or the manufacturer of the device can specify how many samples shall be taken from each source for averaging. For instance, a user can configure the system to take 512 samples from the rms current input, 64 samples from the battery voltage, 64 from the charger input voltage, 32 samples from the external pins and 8 from the NTC pin. Furthermore, the user can also specify if one of the above-mentioned sources shall be skipped.

In some examples, for each source the user can specify two digital thresholds which divide the full range into a plurality of zones, such as 3 zones. Subsequently the user can set the system to release an interrupt when the sampled value changes zones e.g. from a zone 2 to a zone 3.

No conventional IC available in the market today can perform the above features of the PMIC 300. Sampling with such flexibility and granularity is paramount when driving a resonant circuit or component, such as an ultrasound transducer.

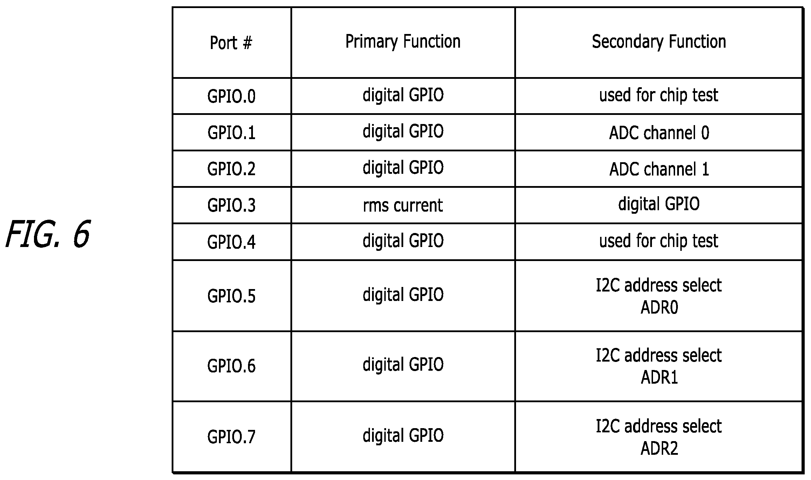

In this example, the PMIC 300 comprises an 8 bit general purpose digital input output port (GPIO). Each port can be configured as digital input and digital output. Some of the ports have an analogue input function, as shown in the table in Figure 6.

The GPIO7-GPIO5 ports of the PMIC 300 can be used to set the device's address on the communication (12C) bus 302. Subsequently eight identical devices can be used on the same I2C bus. This is a unique feature in the IC industry since it allows eight identical devices to be used on the same I2C bus without any conflicting addresses. This is implemented by each device reading the state of GPIO7-GPIO5 during the first 100 µs after the startup of the PMIC 300 and storing that portion of the address internally in the PMIC 300. After the PMIC 300 has been started up the GPIOs can be used for any other purpose.

As described above, the PMIC 300 comprises a six channel LED driver 320. In this example the LED driver 320 comprises N-Channel Metal-Oxide Semiconductor (NMOS) current sources which are 5V tolerant. The LED driver 320 is configured to set the LED current in four discrete levels; 5mA, 10mA, 15mA and 20mA. The LED driver 320 is configured to dim each LED channel with a 12 bit PWM signal either with or without gamma correction. The LED driver 320 is configured to vary the PWM frequency from 300 Hz to 1.5 KHz. This feature is unique in the field of resonant circuit devices, such as ultrasonic mist inhaler devices, as the functionality is embedded as a sub-system of the PMIC 300.

In this example, the PMIC 300 comprises two independent 6 Bit Digital to Analog Converters (DAC) 327, 328 which are incorporated into the PMIC 300. The purpose of the DACs 327, 328 is to output an analogue voltage to manipulate the feedback path of an external regulator (e.g. the DC-DC Boost converter 305 a Buck converter or a LDO). Furthermore, in some examples, the DACs 327, 328 can also be used to dynamically adjust the over current shutdown level of the bridge IC 301, as described below.

The output voltage of each DAC 327, 328 is programmable between 0V and 1.5V or between 0V and V_battery (Vbat). In this example, the control of the DAC output voltage is done via I2C commands. Having two DAC incorporated in the PMIC 300 is unique and will allow the dynamic monitoring control of the current. If either DAC 327, 328 was an external chip, the speed would fall under the same restrictions of speed limitations due to the I2C protocol. An active power monitoring arrangement of the device 202 works with optimum efficiency if all these embedded features are in the PMIC. Had they been external components, the active power monitoring arrangement would be totally inefficient.

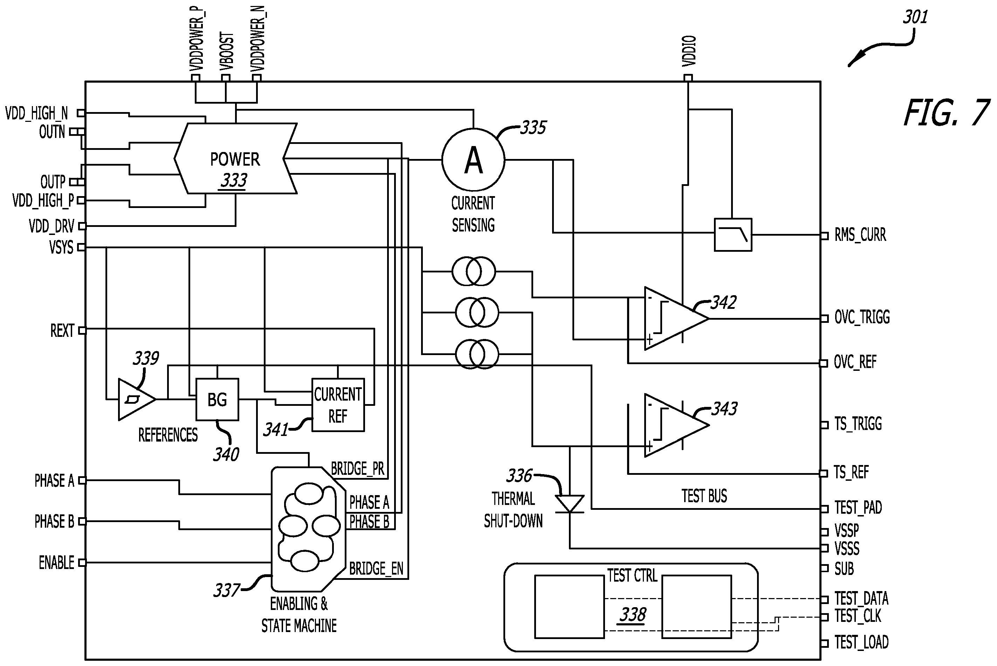

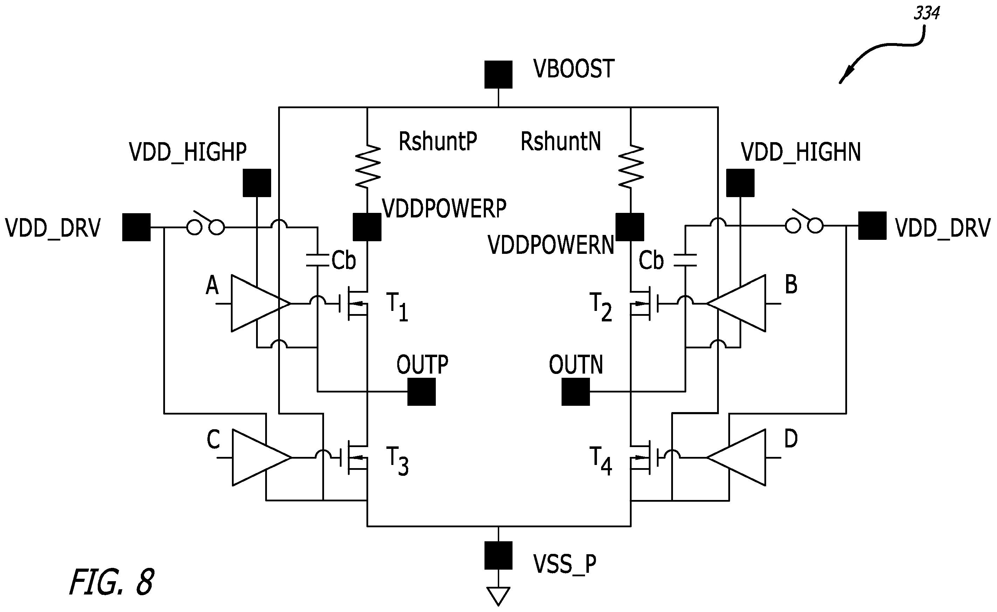

Referring now to Figure 7 of the accompanying drawings, the bridge IC 301 is a microchip which comprises an embedded power switching circuit 333. In this example, the power switching circuit 333 is an H-bridge 334 which is shown in Figure 8 and which is described in detail below. It is, however, to be appreciated that the bridge IC 301 of other examples may incorporate an alternative power switching circuit to the H-bridge 334, provided that the power switching circuit performs an equivalent function for generating an AC drive signal to drive the ultrasonic transducer 215.

The bridge IC 301 comprises a first phase terminal PHASE A which receives a first phase output signal Phase A from the PWM signal generator subsystem of the PMIC 300. The bridge IC 301 also comprises a second phase terminal PHASE B which receives a second phase output signal Phase B from the PWM signal generator subsystem of the PMIC 300.

The bridge IC 301 comprises a current sensing circuit 335 which senses current flow in the H-bridge 334 directly and provides an RMS current output signal via the RMS_CURR pin of the bridge IC 301. The current sensing circuit 335 is configured for over current monitoring, to detect when the current flowing in the H-bridge 334 is above a predetermined threshold. The integration of the power switching circuit 333 comprising the H-bridge 334 and the current sensing circuit 335 all within the same embedded circuit of the bridge IC 301 is a unique combination in the IC market. At present, no other integrated circuit in the IC market comprises an H-bridge with embedded circuitry for sensing the RMS current flowing through the H-bridge.

The bridge IC 301 comprises a temperature sensor 336 which includes over temperature monitoring. The temperature sensor 336 is configured to shut down the bridge IC 301 or disable at least part of the bridge IC 336 in the event that the temperature sensor 336 detects that the bridge IC 301 is operating at a temperature above a predetermined threshold. The temperature sensor 336 therefore provides an integrated safety function which prevents damage to the bridge IC 301 or other components within the driver device 202 in the event that the bridge IC 301 operates at an excessively high temperature.

The bridge IC 301 comprises a digital state machine 337 which is integrally connected to the power switching circuit 333. The digital state machine 337 receives the phase A and phase B signals from the PMIC 300 and an ENABLE signal, for instance from the microcontroller 303. The digital state machine 337 generates timing signals based on the first phase output signal Phase A and the second phase output signal Phase B.

The digital state machine 337 outputs timing signals corresponding to the phase A and phase B signals as well as a BRIDGE PR and BRIDGE EN signals to the power switching circuit 333 in order to control the power switching circuit 333. The digital state machine 337 thus outputs the timing signals to the switches T₁-T₄ of the H-bridge circuit 334 to control the switches T₁-T₄ to turn on and off in a sequence such that the H-bridge circuit outputs an AC drive signal for driving a resonant circuit, such as the ultrasonic transducer 215.

As described in more detail below, the switching sequence comprises a free-float period in which the first switch T₁ and the second switch T₁ are turned off and the third switch T₃ and the fourth switch T₄ are turned on in order to dissipate energy stored by the resonant circuit (the ultrasonic transducer 215).

The bridge IC 301 comprises a test controller 338 which enables the bridge IC 301 to be tested to determine whether the embedded components within the bridge IC 301 are operating correctly. The test controller 338 is coupled to TEST_DATA, TEST_CLK and TEST_LOAD pins so that the bridge IC 301 can be connected to an external control device which feeds data into and out from the bridge IC 301 to test the operation of the bridge IC 301. The bridge IC 301 also comprises a TEST BUS which enables the digital communication bus within the bridge IC 301 to be tested via a TST_PAD pin.

The bridge IC 301 comprises a power on reset circuit (POR) 339 which controls the startup operation of the bridge IC 301. The POR 339 ensures that the bridge IC 301 starts up properly only if the supply voltage is within a predetermined range. If the power supply voltage is outside of the predetermined range, for instance if the power supply voltage is too high, the POR 339 delays the startup of the bridge IC 301 until the supply voltage is within the predetermined range.

The bridge IC 301 comprises a reference block (BG) 340 which provides a precise reference voltage for use by the other subsystems of the bridge IC 301.

The bridge IC 301 comprises a current reference 341 which provides a precise current to the power switching circuit 333 and/or other subsystems within the bridge IC 301, such as the current sensor 335.

The temperature sensor 336 monitors the temperature of the silicon of the bridge IC 301 continuously. If the temperature exceeds the predetermined temperature threshold, the power switching circuit 333 is switched off automatically. In addition, the over temperature may be reported to an external host to inform the external host that an over temperature event has occurred.

The digital state machine (FSM) 337 generates the timing signals for the power switching circuit 333 which, in this example, are timing signals for controlling the H-bridge 334.

The bridge IC 301 comprises comparators 342,343 which compare signals from the various subsystems of the bridge IC 301 with the voltage and current references 340,341 and provide reference output signals via the pins of the bridge IC 301.

Referring again to Figure 8 of the accompanying drawings, the H-bridge 334 of this example comprises four switches in the form of NMOS field effect transistors (FET) switches on both sides of the H-bridge 334. The H-bridge 334 comprises four switches or transistors T₁-T₄ which are connected in an H-bridge configuration, with each transistor T₁-T₄ being driven by a respective logic input A-D. The transistors T₁-T₄ are configured to be driven by a bootstrap voltage which is generated internally with two external capacitors Cb which are connected as illustrated in Figure 8.

The H-bridge 334 comprises various power inputs and outputs which are connected to the respective pins of the bridge IC 301. The H-bridge 334 receives the programmable voltage VBOOST which is output from the boost converter 305 via a first power supply terminal, labelled VBOOST in Figure 8. The H-bridge 334 comprises a second power supply terminal, labelled VSS_P in Figure 8.

The H-bridge 334 comprises outputs OUTP, OUTN which are configured to connect to respective terminals of the ultrasonic transducer 215 so that the AC drive signal output from the H-bridge 334 can drive the ultrasonic transducer 215.

The switching of the four switches or transistors T₁-T₄ is controlled by switching signals from the digital state machine 337 via the logic input A-D. It is to be appreciated that, while Figure 8 shows four transistors T₁-T₄, in other examples, the H-bridge 334 incorporates a larger number of transistors or other switching components to implement the functionality of the H-bridge.

In this example, the H-bridge 334 operates at a switching power of 22 W to 50 W in order to deliver an AC drive signal with sufficient power to drive the ultrasonic transducer 215 optimally at or near the resonant frequency of the ultrasonic transducer 215. The voltage which is switched by the H-bridge 334 of this example is ±15 V. In other examples, the voltage is ±20 V.

In this example, the H-bridge 334 switches at a frequency of 3 MHz to 5 MHz or up to 105 MHz. This is a high switching speed compared with conventional integrated circuit H-bridges which are available in the IC market. For instance, a conventional integrated circuit H-bridge available in the IC market today is configured to operate at a maximum frequency of only 2 MHz. Aside from the bridge IC 301 described herein, no conventional integrated circuit H-bridge available in the IC market is able to operate at a power of 22 V to 50 V at a frequency of up to 5 MHz, let alone up to 105 MHz.

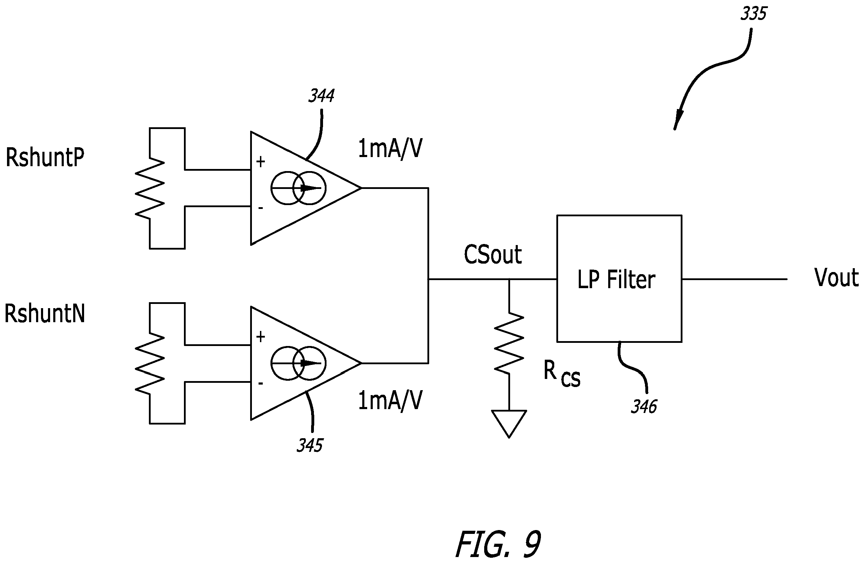

Referring now to Figure 9 of the accompanying drawings, the current sensor 335 comprises positive and negative current sense resistors RshuntP, RshuntN which are connected in series with the respective high and low sides of the H-bridge 334, as shown in Figure 8. The current sense resistors RshuntP, RshuntN are low value resistors which, in this example, are 0.1 Ω. The current sensor 335 comprises a first voltage sensor in the form of a first operational amplifier 344 which measures the voltage drop across the first current sensor resistor RshuntP and a second voltage sensor in the form of a second operational amplifier 345 which measures the voltage drop across the second current sensor resistor RshuntN. In this example, the gain of each operational amplifier 344, 345 is 2V/V. The output of each operational amplifier 344, 345 is, in this example, 1mA/V. The current sensor 335 comprises a pull down resistor Rcs which, in this example, is 2kΩ. The outputs of the operational amplifiers 344, 345 provide an output CSout which passes through a low pass filter 346 which removes transients in the signal CSout. An output Vout of the low pass filter 346 is the output signal of the current sensor 335.

The current sensor 335 thus measures the AC current flowing through the H-bridge 334 and respectively through the ultrasonic transducer 215. The current sensor 335 translates the AC current into an equivalent RMS output voltage (Vout) relative to ground. The current sensor 335 has high bandwidth capability since the H-bridge 334 can be operated at a frequency of up to 5 MHz or, in some examples, up to 105 MHz. The output Vout of the current sensor 335 reports a positive voltage which is equivalent to the measured AC rms current flowing through the ultrasonic transducer 215. The output voltage Vout of the current sensor 335 is, in this example, fed back to the control circuitry within the bridge IC 301 to enable the bridge IC 301 to shut down the H-bridge 334 in the event that the current flowing through the H-bridge 334 and hence through the transducer 215 is in excess of a predetermined threshold. In addition, the over current threshold event is reported to the first comparator 342 in the bridge IC 301 so that the bridge IC 301 can report the over current event via the OVC_TRIGG pin of the bridge IC 301.

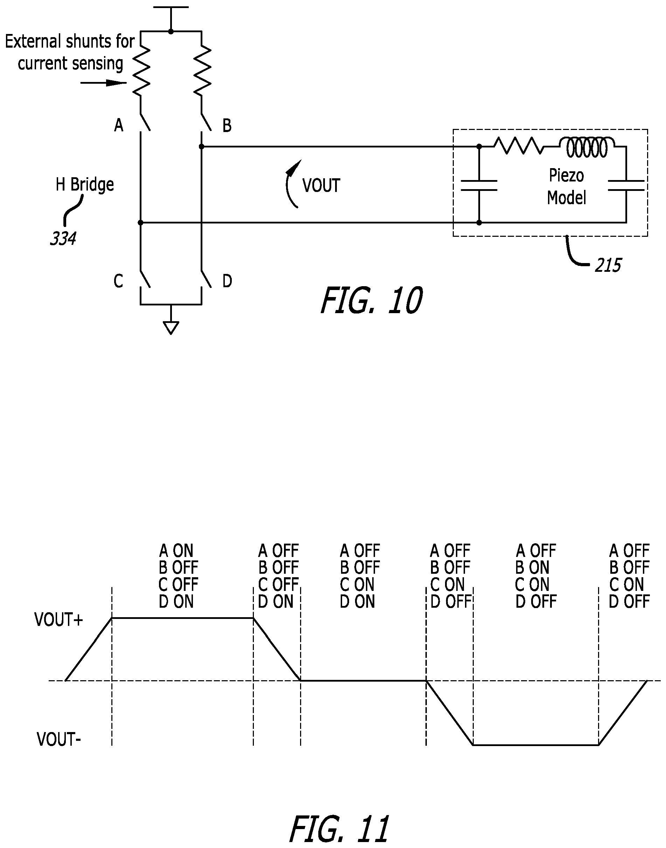

Referring now to Figure 10 of the accompanying drawings, the control of the H-bridge 334 will now be described also with reference to the equivalent piezoelectric model of the ultrasonic transducer 215.

To develop a positive voltage across the outputs OUTP, OUTN of the H-bridge 334 as indicated by V_out in Figure 10 (note the direction of the arrow) the switching sequence of the transistors T₁-T₄ via the inputs A-D is as follows:
1. Positive output voltage across the ultrasonic transducer 215: A-ON, B-OFF, C-OFF, D-ON
2. Transition from positive output voltage to zero: A-OFF, B-OFF, C-OFF, DON. During this transition, C is switched off first to minimise or avoid power loss by minimising or avoiding current flowing through A and C if there is a switching error or delay in A.
3. Zero output voltage: A-OFF, B-OFF, C-ON, D-ON. During this zero output voltage phase, the terminals of the outputs OUTP, OUTN of the H-bridge 334 are grounded by the C and D switches which remain on. This dissipates the energy stored by the capacitors in the equivalent circuit of the ultrasonic transducer, which minimises the voltage overshoot in the switching waveform voltage which is applied to the ultrasonic transducer.
4. Transition from zero to negative output voltage: A-OFF, B-OFF, C-ON, D-OFF.
5. Negative output voltage across the ultrasonic transducer 215: A-OFF, B-ON, C-ON, D-OFF

At high frequencies of up to 5 MHz or even up to 105 MHz, it will be appreciated that the time for each part of the switching sequence is very short and in the order of nanoseconds or picoseconds. For instance, at a switching frequency of 6 MHz, each part of the switching sequence occurs in approximately 80 ns.

A graph showing the output voltage OUTP, OUTN of the H-bridge 334 according to the above switching sequence is shown in Figure 11 of the accompanying drawings. The zero output voltage portion of the switching sequence is included to accommodate for the energy stored by the ultrasonic transducer 215 (e.g. the energy stored by the capacitors in the equivalent circuit of the ultrasonic transducer). As described above, this minimises the voltage overshoot in the switching waveform voltage which is applied to the ultrasonic transducer and hence minimises unnecessary power dissipation and heating in the ultrasonic transducer.

Minimising or removing voltage overshoot also reduces the risk of damage to transistors in the bridge IC 301 by preventing the transistors from being subject to voltages in excess of their rated voltage. Furthermore, the minimisation or removal of the voltage overshoot enables the bridge IC 301 to drive the ultrasonic transducer accurately in a way which minimises disruption to the current sense feedback loop described herein. Consequently, the bridge IC 301 is able to drive the ultrasonic transducer at a high power of 22 W to 50 W or even as high as 70 W at a high frequency of up to 5 MHz or even up to 105 MHz.

The bridge IC 301 of this example is configured to be controlled by the PMIC 300 to operate in two different modes, referred to herein as a forced mode and a native frequency mode. These two modes of operation are novel over existing bridge ICs. In particular, the native frequency mode is a major innovation which offers substantial benefits in the accuracy and efficiency of driving an ultrasonic transducer as compared with conventional devices.

### Forced Frequency Mode (FFM)

In the forced frequency mode the H-bridge 334 is controlled in the sequence described above but at a user selectable frequency. As a consequence, the H-bridge transistors T₁-T₄ are controlled in a forced way irrespective of the inherent resonant frequency of the ultrasonic transducer 215 to switch the output voltage across the ultrasonic transducer 215. The forced frequency mode therefore allows the H-bridge 334 to drive the ultrasonic transducer 215, which has a resonant frequency f1, at different frequency f2.

Driving an ultrasonic transducer at a frequency which is different from its resonant frequency may be appropriate in order to adapt the operation to different applications. For example, it may be appropriate to drive an ultrasonic transducer at a frequency which is slightly off the resonance frequency (for mechanical reasons to prevent mechanical damage to the transducer). Alternatively, it may be appropriate to drive an ultrasonic transducer at a low frequency but the ultrasonic transducer has, because of its size, a different native resonance frequency.

The driver device 202 controls the bridge IC 301 to drive the ultrasonic transducer 215 in the forced frequency mode in response to the configuration of the driver device 202 for a particular application or a particular ultrasonic transducer. For instance, the driver device 202 may be configured to operate in the forced frequency mode when the resonant circuit device 201 is being used for a particular application, such as generating a mist from a liquid of a particular viscosity containing a drug for delivery to a user.

### Native Frequency Mode (NFM)

The following native frequency mode of operation is a significant development and provides benefits in improved accuracy and efficiency over conventional ultrasonic drivers that are available on the IC market today.

The native frequency mode of operation follows the same switching sequence as described above but the timing of the zero output portion of the sequence is adjusted to minimise or avoid problems that can occur due to current spikes in the forced frequency mode operation. These current spikes occur when the voltage across the ultrasonic transducer 215 is switched to its opposite voltage polarity.

An ultrasonic transducer which comprises a piezoelectric crystal has an electrical equivalent circuit which incorporates a parallel connected capacitor (e.g. see the piezo model in Figure 10). If the voltage across the ultrasonic transducer is hard-switched from a positive voltage to a negative voltage, due to the high dV/dt there can be a large current flow current flow as the energy stored in the capacitor dissipates.

The native frequency mode avoids hard switching the voltage across the ultrasonic transducer 215 from a positive voltage to a negative voltage (and vice versa). Instead, prior to applying the reversed voltage, the ultrasonic transducer 215 (piezoelectric crystal) is left free-floating with zero voltage applied across its terminals for a free-float period. The PMIC 300 sets the drive frequency of the bridge IC 301 such that the bridge 334 sets the free-float period such that current flow inside the ultrasonic transducer 215 (due to the energy stored within the piezoelectric crystal) reverses the voltage across the terminals of the ultrasonic transducer 215 during the free-float period.

Consequently, when the H-bridge 334 applies the negative voltage at the terminals of the ultrasonic transducer 215 the ultrasonic transducer 215 (the capacitor in the equivalent circuit) has already been reverse charged and no current spikes occur because there is no high dV/dt.

It is, however, to be appreciated that it takes time for the charge within the ultrasonic transducer 215 (piezoelectric crystal) to build up when the ultrasonic transducer 215 is first activated. Therefore, the ideal situation in which the energy within the ultrasonic transducer 215 is to reverse the voltage during the free-float period occurs only after the oscillation inside the ultrasonic transducer 215 has built up the charge. To accommodate for this, when the bridge IC 301 activates the ultrasonic transducer 215 for the first time, the PMIC 300 controls the power delivered through the H-bridge 334 to the ultrasonic transducer 215 to a first value which is a low value (e.g. 5 V). The PMIC 300 then controls the power delivered through the H-bridge 334 to the ultrasonic transducer 215 to increase over a period of time to a second value (e.g. 15 V) which is higher than the first value in order to build up the energy stored within the ultrasonic transducer 215. Current spikes still occur during this ramp of the oscillation until the current inside the ultrasonic transducer 215 developed sufficiently. However, by using a low first voltage at start up those current spikes are kept sufficiently low to minimise the impact on the operation of the ultrasonic transducer 215.

In order to implement the native frequency mode, the driver device 202 controls the frequency of the oscillator 315 and the duty cycle (ratio of turn-on time to free-float time) of the AC drive signal output from the H-bridge 334 with high precision. In this example, the driver device 202 performs three control loops to regulate the oscillator frequency and the duty cycle such that the voltage reversal at the terminals of the ultrasonic transducer 215 is as precise as possible and current spikes are minimised or avoided as far as possible. The precise control of the oscillator and the duty cycle using the control loops is a significant advance in the field of IC ultrasonic drivers.

During the native frequency mode of operation, the current sensor 335 senses the current flowing through the ultrasonic transducer 215 (resonant circuit) during the free-float period. The digital state machine 337 adapts the timing signals to switch on either the first switch T1 or the second switch T2 when the current sensor 335 senses that the current flowing through the ultrasonic transducer 215 (resonant circuit) during the free-float period is zero.

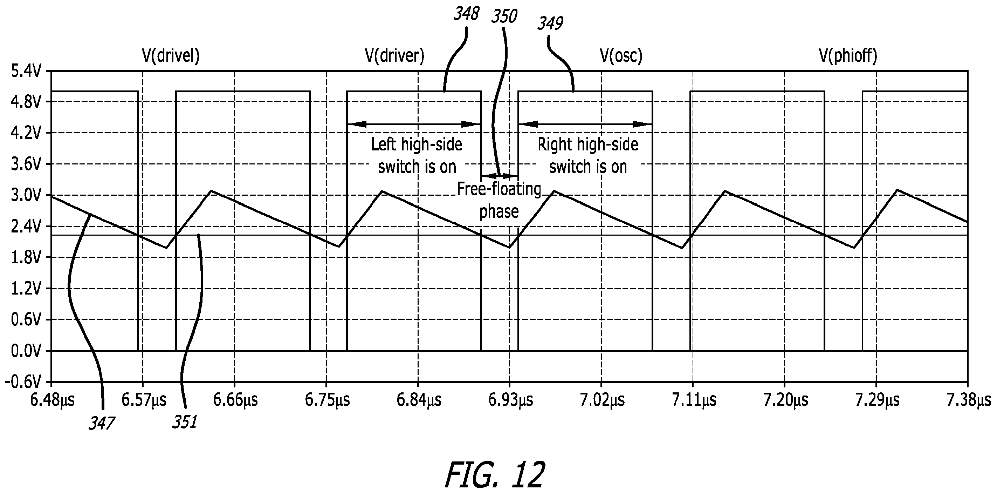

Figure 12 of the accompanying drawings shows the oscillator voltage waveform 347 (V(osc)), a switching waveform 348 resulting from the turn-on and turn-off the left hand side high switch T1 of the H-bridge 334 and a switching waveform 349 resulting from the turn-on and turn-off the right hand side high switch T2 of the H-bridge 334. For an intervening free-float period 350, both high switches T1, T2 of the H-bridge 334 are turned off (free-floating phase). The duration of the free-float period 350 is controlled by the magnitude of the free-float control voltage 351 (Vphioff).

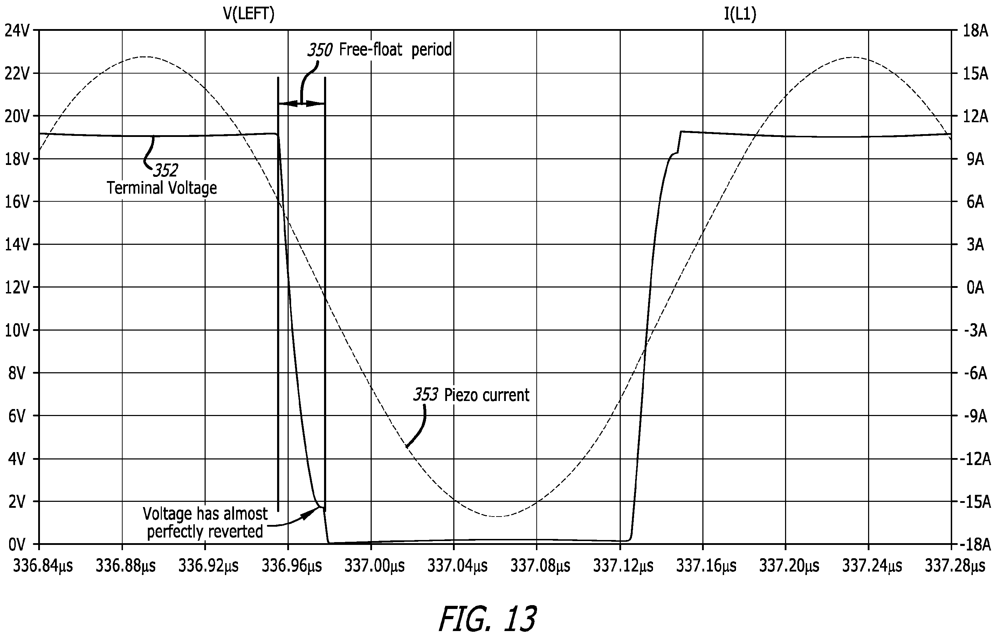

Figure 13 of the accompanying drawings shows the voltage waveform 352 at a first terminal of the ultrasonic transducer 215 (the voltage waveform is reversed at the second terminal of the ultrasonic transducer 215) and the piezo current 353 flowing through the ultrasonic transducer 215. The piezo current 353 represents an (almost) ideal sinusoidal waveform (this is never possible in the forced frequency mode or in any bridge in the IC market).

Before the sinusoidal wave of the piezo current 353 reaches zero, the left hand side high switch T1 of the H-bridge 334 is turned off (here, the switch T1 is turned off when the piezo current 353 is approximately 6 A). The remaining piezo current 353 which flows within the ultrasonic transducer 215 due to the energy stored in the ultrasonic transducer 215 (the capacitor of the piezo equivalent circuit) is responsible for the voltage reversal during the free-float period 350. The piezo current 353 decays to zero during the free-float period 350 and into negative current flow domain thereafter. The terminal voltage at the ultrasonic transducer 215 drops from the supply voltage (in this case 19 V) to less than 2 V and the drop comes to a stop when the piezo current 353 reaches zero. This is the perfect time to turn on the low-side switch T3 of the H-bridge 334 in order to minimise or avoid a current spike.

Compared to the forced frequency mode described above, the native frequency mode has at least three advantages:
1. The current spike associated with hard switching of the package capacitor is significantly reduced or avoided completely.
2. Power loss due to hard switching is almost eliminated.
3. Frequency is regulated by the control loops and will be kept close to the resonance of the piezo crystal (i.e. the native resonance frequency of the piezo crystal).

In the case of the frequency regulation by the control loops (advantage 3 above), the PMIC 300 starts by controlling the bridge IC 301 to drive the ultrasonic transducer 215 at a frequency above the resonance of the piezo crystal. The PMIC 300 then controls the bridge IC 301 to that the frequency of the AC drive signal decays/reduces during start up. As soon as the frequency approaches resonance frequency of the piezo crystal, the piezo current will develop/increase rapidly. Once the piezo current is high enough to cause the desired voltage reversal, the frequency decay/reduction is stopped by the PMIC 300. The control loops of the PMIC 300 then take over the regulation of frequency and duty cycle of the AC drive signal.

In the forced frequency mode, the power delivered to the ultrasonic transducer 215 is controlled through the duty cycle and/or a frequency shift and/or by varying the supply voltage. However, in this example in the native frequency mode the power delivered to the ultrasonic transducer 215 controlled only through the supply voltage.

In this example, during a setup phase of operation of the driver device, the bridge IC 301 is configured to measure the length of time taken for the current flowing through the ultrasonic transducer 215 (resonant circuit) to fall to zero when the first switch T₁ and the second switch T₂ are turned off and the third switch T₃ and the fourth switch T₄ are turned on. The bridge IC 301 then sets the length of time of the free-float period to be equal to the measured length of time.

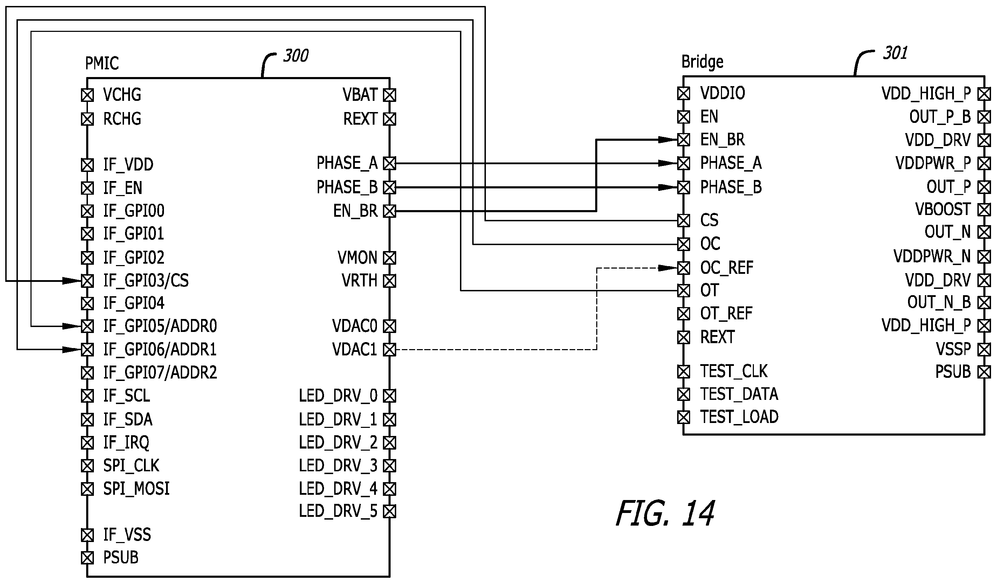

Referring now to Figure 14 of the accompanying drawings, the PMIC 300 and the bridge IC 301 of this example are designed to work together as a companion chip set. The PMIC 300 and the bridge IC 301 are connected together electrically for communication with one another. In this example, there are interconnections between the PMIC 300 and the bridge IC 301 which enable the following two categories of communication:
1. control signals
2. feedback signals

The connections between the PHASE_A and PHASE_B pins of the PMIC 300 and the bridge IC 301 carry the PWM modulated control signals which drive the H-bridge 334. The connection between the EN_BR pins of the PMIC 300 and the bridge IC 301 carries the EN_BR control signal which triggers the start of the H-bridge 334. The timing between the PHASE_A, PHASE_B and EN_BR control signals is important and handled by the digital bridge control of the PMIC 300.

The connections between the CS, OC and OT pins of the PMIC 300 and the bridge IC 301 carry CS (current sense), OC (over current) and OT (over temperature) feedback signals from the bridge IC 301 back to the PMIC 300. Most notably, the CS (current sense) feedback signal comprises a voltage equivalent to the rms current flowing through the ultrasonic transducer 215 which is measured by the current sensor 335 of the bridge IC 301.

The OC (over current) and OT (over temperature) feedback signals are digital signals indicating that either an over current or an over voltage event has been detected by the bridge IC 301. In this example, the thresholds for the over current and over temperature are set with an external resistor. Alternatively, the thresholds can also be dynamically set in response to signals passed to the OC_REF pin of the bridge IC 301 from one of the two DAC channels VDAC0, VDAC1 from the PMIC 300.

In this example, the design of the PMIC 300 and the bridge IC 301 allow the pins of these two integrated circuits to be connected directly to one another (e.g. via copper tracks on a PCB) so that there is minimal or no lag in the communication of signals between the PMIC 300 and the bridge IC 301. This provides a significant speed advantage over conventional bridges in the IC market which are typically controlled by signals via a digital communications bus. For example, a standard I2C bus is clocked at only 400 kHz, which is too slow for communicating data sampled at the high clock speeds of up to 5 MHz of examples of this disclosure.

While examples of this disclosure have been described above in relation to the microchip hardware, it is to be appreciated that other examples of this disclosure comprise a method of operating the components and subsystems of each microchip to perform the functions described herein. For instance, the methods of operating the PMIC 300 and the bridge IC 301 in either the forced frequency mode or the native frequency mode.

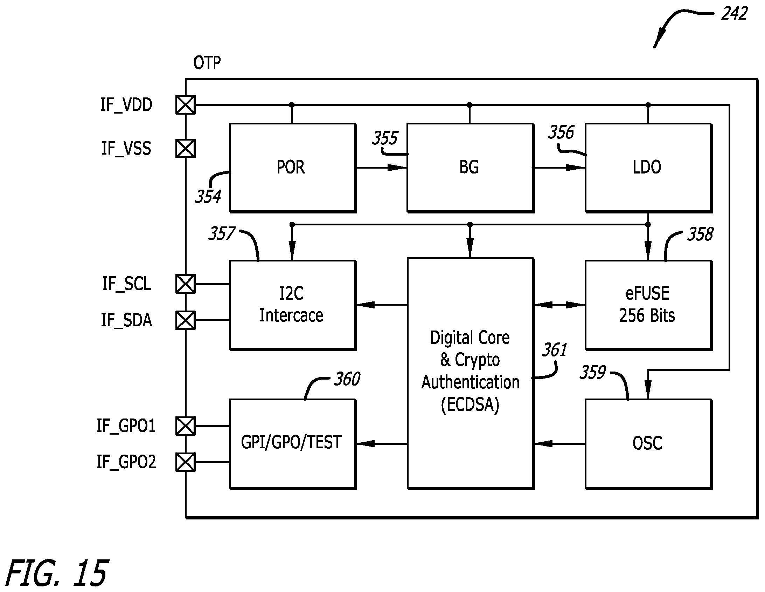

Referring now to Figure 15 of the accompanying drawings, the optional OTP IC 242 comprises a power on reset circuit (POR) 354, a bandgap reference (BG) 355, a cap-less low dropout regulator (LDO) 356, a communication (e.g. I2C ) interface 357, a one-time programmable memory bank (eFuse) 358, an oscillator 359 and a general purpose input-output interface 360. The OTP IC 242 also comprises a digital core 361 which includes a cryptographic authenticator. In this example, the cryptographic authenticator uses the Elliptic Curve Digital Signature Algorithm (ECDSA) for encrypting/decrypting data stored within the OTP IC 242 as well as data transmitted to and from the OTP IC 242.

The POR 354 ensures that the OTP IC 242 starts up properly only if the supply voltage is within a predetermined range. If the supply voltage is outside the predetermined range, the POR 354 resets the OTP IC 242 and waits until the supply voltage is within the predetermined range.

The BG 355 provides precise reference voltages and currents to the LDO 356 and to the oscillator 359. The LDO 356 supplies the digital core 361, the communication interface 357 and the eFuse memory bank 358.

The OTP IC 242 is configured to operate in at least the following modes:
- Fuse Programming (Fusing): During efuse programming (programming of the one time programmable memory) a high current is required to burn the relevant fuses within the eFuse memory bank 358. In this mode higher bias currents are provided to maintain gain and bandwidth of the regulation loop.
- Fuse Reading: In this mode a medium level current is required to maintain efuse reading within the eFuse memory bank 358. This mode is executed during the startup of the OTP IC 242 to transfer the content of the fuses to shadow registers. In this mode the gain and bandwidth of the regulation loop is set to a lower value than in the Fusing Mode.
- Normal Operation: In this mode the LDO 356 is driven in a very low bias current condition to operate the OTP IC 242 with low power so that the OTP IC 242 consumes as little power as possible.

The oscillator 359 provides the required clock for the digital core/engine 361 during testing (SCAN Test), during fusing and during normal operation. The oscillator 359 is trimmed to cope with the strict timing requirements during the fusing mode.

In this example, the communication interface 357 is compliant with the FM+ specification of the I2C standard but it also complies with slow and fast mode. The OTP IC 242 uses the communication interface 357 to communicate with the driver device 202 (the Host) for data and key exchange.

The digital core 361 implements the control and communication functionality of the OTP IC 242. The cryptographic authenticator of the digital core 361 enables the OTP IC 242 to authenticate itself (e.g. using ECDSA encrypted messages) with the driver device 202 (e.g. for a particular application) to ensure that the OTP IC 242 is genuine and that the OTP IC 242 is authorised to connect to the driver device 202 (or another product).

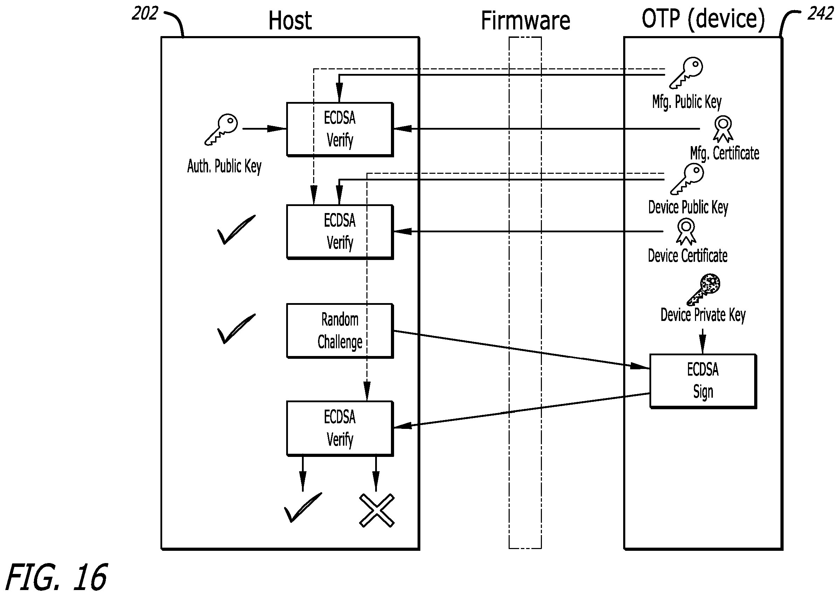

With reference to Figure 16 of the accompanying drawings, the OTP IC 242 performs the following PKI procedure in order to authenticate the OTP IC 242 for use with a Host (e.g. the driver device 202):
1. Verify Signer Public Key: The Host requests the Manufacturing Public key and Certificate. The Host verifies the certificate with the Authority Public key.
2. Verify Device Public Key: If the verification is successful, the Host requests the Device Public key and Certificate. The Host verifies the certificate with the Manufacturing Public key.
3. Challenge - Response: If the verification is successful, the Host creates a random number challenge and sends it to the Device. The End Product signs the random number challenge with the Device Private key.
4. The signature is sent back to the Host for verification using the Device Public key.

If all steps of the authentication procedure complete successfully then the Chain of Trust has been verified back to the Root of Trust and the OTP IC 242 is successfully authenticated for use with the Host. However, if any of the steps of the authentication procedure fail then the OTP IC 242 is not authenticated for use with the Host and use of the device incorporating the OTP IC 242 is restricted or prevented.

The foregoing outlines features of several examples or embodiments so that those of ordinary skill in the art may better understand various aspects of the present disclosure. Those of ordinary skill in the art should appreciate that they may readily use the present disclosure as a basis for designing or modifying other processes and structures for carrying out the same purposes and/or achieving the same advantages of various examples or embodiments introduced herein. Those of ordinary skill in the art should also realise that such equivalent constructions do not depart from the spirit and scope of the present disclosure, and that they may make various changes, substitutions, and alterations herein without departing from the spirit and scope of the present disclosure.

Although the subject matter has been described in language specific to structural features or methodological acts, it is to be understood that the subject matter of the appended claims is not necessarily limited to the specific features or acts described above. Rather, the specific features and acts described above are disclosed as example forms of implementing at least some of the claims.

Various operations of examples or embodiments are provided herein. The order in which some or all of the operations are described should not be construed to imply that these operations are necessarily order dependent. Alternative ordering will be appreciated having the benefit of this description. Further, it will be understood that not all operations are necessarily present in each embodiment provided herein. Also, it will be understood that not all operations are necessary in some examples or embodiments.

Moreover, "exemplary" is used herein to mean serving as an example, instance, illustration, etc., and not necessarily as advantageous. As used in this application, "or" is intended to mean an inclusive "or" rather than an exclusive "or". In addition, "a" and "an" as used in this application and the appended claims are generally be construed to mean "one or more" unless specified otherwise or clear from context to be directed to a singular form. Also, at least one of A and B and/or the like generally means A or B or both A and B. Furthermore, to the extent that "includes", "having", "has", "with", or variants thereof are used, such terms are intended to be inclusive in a manner similar to the term "comprising". Also, unless specified otherwise, "first," "second," or the like are not intended to imply a temporal aspect, a spatial aspect, an ordering, etc. Rather, such terms are merely used as identifiers, names, etc. for features, elements, items, etc. For example, a first element and a second element generally correspond to element A and element B or two different or two identical elements or the same element.

Also, although the disclosure has been shown and described with respect to one or more implementations, equivalent alterations and modifications will occur to others of ordinary skill in the art based upon a reading and understanding of this specification and the annexed drawings. The disclosure comprises all such modifications and alterations and is limited only by the scope of the following claims. In particular regard to the various functions performed by the above described features (e.g., elements, resources, etc.), the terms used to describe such features are intended to correspond, unless otherwise indicated, to any features which performs the specified function of the described features (e.g., that is functionally equivalent), even though not structurally equivalent to the disclosed structure. In addition, while a particular feature of the disclosure may have been disclosed with respect to only one of several implementations, such feature may be combined with one or more other features of the other implementations as may be desired and advantageous for any given or particular application.

Examples or embodiments of the subject matter and the functional operations described herein can be implemented in digital electronic circuitry, or in computer software, firmware, or hardware, including the structures disclosed in this specification and their structural equivalents, or in combinations of one or more of them.

Some examples or embodiments are implemented using one or more modules of computer program instructions encoded on a computer-readable medium for execution by, or to control the operation of, a data processing apparatus. The computer-readable medium can be a manufactured product, such as hard drive in a computer system or an embedded system. The computer-readable medium can be acquired separately and later encoded with the one or more modules of computer program instructions, such as by delivery of the one or more modules of computer program instructions over a wired or wireless network. The computer-readable medium can be a machine-readable storage device, a machine-readable storage substrate, a memory device, or a combination of one or more of them.

The terms "computing device" and "data processing apparatus" encompass all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, a runtime environment, or a combination of one or more of them. In addition, the apparatus can employ various different computing model infrastructures, such as web services, distributed computing and grid computing infrastructures.

The processes and logic flows described in this specification can be performed by one or more programmable processors executing one or more computer programs to perform functions by operating on input data and generating output. Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Devices suitable for storing computer program instructions and data include all forms of non-volatile memory, media and memory devices.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

## Claims

1. An apparatus for transmitting ultrasonic waves, the apparatus comprising:
a resonant circuit, wherein the resonant circuit is at least one of an LC tank, an antenna and a piezoelectric transducer;
an H-bridge circuit (334) connected to the resonant circuit, wherein the H-bridge circuit (334) is configured to generate an AC drive signal to drive the resonant circuit to generate and transmit the ultrasonic waves;
a microchip connected to the H-bridge circuit (334), wherein the microchip is configured to control the H-bridge circuit (334) to generate the AC drive signal, wherein the microchip is a single unit which comprises a plurality of interconnected embedded components and subsystems comprising:
an oscillator (315) which is configured to generate:
a main clock signal,
a first phase clock signal which is high for a first time during the positive half-period of the main clock signal and low during the negative half-period of the main clock signal, and
a second phase clock signal which is high for a second time during the negative half-period of the main clock signal and low during the positive half-period of the main clock signal, wherein the phases of the first phase clock signal and the second phase clock signal are centre aligned;
a pulse width modulation (PWM) signal generator subsystem (329) comprising:
a delay locked loop (332) which is configured to generate a double frequency clock signal using the first phase clock signal and the second phase clock signal, the double frequency clock signal being double the frequency of the main clock signal, wherein the delay locked loop (332) is configured to control the rising edge of the first phase clock signal and the second phase clock signal to be synchronous with the rising edge of the double frequency clock signal, and wherein the delay locked loop (332) is configured to adjust the frequency and the duty cycle of the first phase clock signal and the second phase clock signal in response to a driver control signal to produce a first phase output signal and a second phase output signal, wherein the first phase output signal and the second phase output signal are configured to drive the H-bridge circuit (334) to generate an AC drive signal to drive the resonant circuit;
a first phase output signal terminal which is configured to output the first phase output signal to the H-bridge circuit (334);
a second phase output signal terminal which is configured to output the second phase output signal to the H-bridge circuit (334);
a feedback input terminal which is configured to receive a feedback signal from the H-bridge circuit (334), the feedback signal being indicative of a parameter of the operation of the H-bridge circuit (334) or the AC drive signal when the H-bridge circuit (334) is driving the resonant circuit with the AC drive signal;
an analogue to digital converter (ADC) subsystem (318) comprising:
a plurality of ADC input terminals (319) which are configured to receive a plurality of respective analogue signals, wherein one ADC input terminal of the plurality of ADC input terminals (319) is connected to the feedback input terminal such that the ADC subsystem (318) receives the feedback signal from the H-bridge circuit (334), and wherein the ADC subsystem (318) is configured to sample analogue signals received at the plurality of ADC input terminals (319) at a sampling frequency which is proportional to the frequency of the main clock signal and the ADC subsystem (318) is configured to generate ADC digital signals using the sampled analogue signals;
a digital processor subsystem which is configured to receive the ADC digital signals from the ADC subsystem (318) and process the ADC digital signals to generate the driver control signal, wherein the digital processor subsystem is configured to communicate the driver control signal to the **PWM** signal generator subsystem to control the **PWM** signal generator subsystem (329); and
a digital to analogue converter (DAC) subsystem comprising:
a digital to analogue converter (DAC) (327) which is configured to convert a digital control signal generated by the digital processor subsystem into an analogue voltage control signal to control a voltage regulator circuit which is configured to generate a voltage for modulation by the H-bridge circuit (334); and
a DAC output terminal which is configured to output the analogue voltage control signal to control the voltage regulator circuit to generate a predetermined voltage for modulation by the H-bridge circuit (334) to drive a resonant circuit in response to feedback signals which are indicative of the operation of the resonant circuit.

2. The apparatus of claim 1, wherein the oscillator (315) is configured to generate the main clock signal at a frequency of 50 kHz to 105 MHz.

3. The apparatus of claim 1 or claim 2, wherein the microchip further comprises:
a frequency divider which is connected to the oscillator (315) to receive the main clock signal from the oscillator (315), the frequency divider being configured to divide the main clock signal by a predetermined divisor amount and output the frequency reference signal to the delay locked loop (332).

4. The apparatus of any one of the preceding claims, wherein the delay locked loop (332) comprises a plurality of delay lines connected end to end, wherein the total delay of the delay lines is equal to the period of the main clock signal.

5. The apparatus of claim 4, wherein the delay locked loop (332) is configured to adjust the duty cycle of the first phase clock signal and the second phase clock signal in response to the driver control signal by varying the delay of each delay line in the delay locked loop (332).

6. The apparatus of any one of the preceding claims, wherein the feedback input terminal is configured to receive a feedback signal from the H-bridge circuit (334) in the form of a voltage which indicative of an rms current of the AC drive signal which is driving the resonant circuit.

7. The apparatus of any one of the preceding claims, wherein the ADC subsystem (318) comprises a plurality of further ADC input terminals (319) which are configured to receive feedback signals which are indicative of at least one of the voltage of a battery connected to the apparatus or the voltage of a battery charger connected to the apparatus.

8. The apparatus of any one of the preceding claims, wherein the microchip further comprises:
a temperature sensor which is embedded within the microchip, wherein the temperature sensor is configured to generate a temperature signal which is indicative of the temperature of the microchip, and wherein the temperature signal is received by a further ADC input terminal of the ADC subsystem (318) and the temperature signal is sampled by the ADC.

9. The apparatus of any one of the preceding claims, wherein the ADC subsystem (318) is configured to sample signals received at the plurality of ADC input terminals (319) sequentially with each signal being sampled by the ADC subsystem (318) a respective predetermined number of times.

10. The apparatus of any one of the preceding claims, wherein the microchip further comprises:
a battery charging subsystem which is configured to control the charging of an external battery which is connected to the microchip.

11. The apparatus of any one of the preceding claims, wherein the DAC subsystem comprises:
a further digital to analogue converter (DAC) (328) which is configured to convert a further digital control signal generated by the digital processor subsystem into a further analogue voltage control signal to control the voltage regulator circuit.

## Patentansprüche

1. Einrichtung zum Übertragen von Ultraschallwellen, die Einrichtung umfassend:
einen Resonanzkreis, wobei der Resonanzkreis mindestens eines von einem LC-Tank, einer Antenne und einem piezoelektrischen Wandler ist;
eine H-Brückenschaltung (334), die mit dem Resonanzkreis verbunden ist, wobei die H-Brückenschaltung (334) konfiguriert ist, um ein Wechselstromantriebssignal zu erzeugen, um den Resonanzkreis anzutreiben, um die Ultraschallwellen zu erzeugen und zu übertragen;
einen Mikrochip, der mit der H-Brückenschaltung (334) verbunden ist, wobei der Mikrochip konfiguriert ist, um die H-Brückenschaltung (334) zu steuern, um das Wechselstromantriebssignal zu erzeugen, wobei der Mikrochip eine einzelne Einheit ist, die eine Vielzahl von miteinander verbundenen eingebetteten Komponenten und Subsystemen umfasst, umfassend:
einen Oszillator (315), der konfiguriert ist, um zu erzeugen:
ein Haupttaktsignal,
ein erstes Phasentaktsignal, das während der positiven Halbperiode des Haupttaktsignals ein erstes Mal hoch und während der negativen Halbperiode des Haupttaktsignals niedrig ist, und
ein zweites Phasentaktsignal, das während der negativen Halbperiode des Haupttaktsignals ein zweites Mal hoch und während der positiven Halbperiode des Haupttaktsignals niedrig ist, wobei die Phasen des ersten Phasentaktsignals und des zweiten Phasentaktsignals mittig ausgerichtet sind;
ein Pulsweitenmodulationssignalgeneratorsubsystem (PWM-Signalgeneratorsubsystem) (329), umfassend:
eine Verzögerungsregelschleife (332), die konfiguriert ist, um unter Verwendung des ersten Phasentaktsignals und des zweiten Phasentaktsignals ein Doppelfrequenztaktsignal zu erzeugen, wobei das Doppelfrequenztaktsignal das Doppelte der Frequenz des Haupttaktsignals ist, wobei die Verzögerungsregelschleife (332) konfiguriert ist, um die steigende Flanke des ersten Phasentaktsignals und des zweiten Phasentaktsignals zu steuern, um synchron zu der steigenden Flanke des Doppelfrequenztaktsignals zu sein, und wobei die Verzögerungsregelschleife (332) konfiguriert ist, um die Frequenz und das Tastverhältnis des ersten Phasentaktsignals und des zweiten Phasentaktsignals als Reaktion auf ein Treibersteuersignal anzupassen, um ein erstes Phasenausgangssignal und ein zweites Phasenausgangssignal zu produzieren, wobei das erste Phasenausgangssignal und das zweite Phasenausgangssignal konfiguriert sind, um die H-Brückenschaltung (334) anzutreiben, um ein Wechselstromantriebssignal zu erzeugen, um den Resonanzkreis anzutreiben;
einen ersten Phasenausgangssignalanschluss, der konfiguriert ist, um das erste Phasenausgangssignal an die H-Brückenschaltung (334) auszugeben;
einen zweiten Phasenausgangssignalanschluss, der konfiguriert ist, um das zweite Phasenausgangssignal an die H-Brückenschaltung (334) auszugeben;
einen Rückkopplungseingangsanschluss, der konfiguriert ist, um ein Rückkopplungssignal von der H-Brückenschaltung (334) zu empfangen, wobei das Rückkopplungssignal einen Parameter des Betriebs der H-Brückenschaltung (334) oder das Wechselstromantriebssignal angibt, wenn die H-Brückenschaltung (334) den Resonanzkreis mit dem Wechselstromantriebssignal antreibt;
ein Analog-Digital-Umsetzersubsystem (ADC-Subsystem) (318), umfassend:
eine Vielzahl von ADC-Eingangsanschlüssen (319), die konfiguriert sind, um eine Vielzahl von jeweiligen analogen Signalen zu empfangen, wobei ein ADC-Eingangsanschluss der Vielzahl von ADC-Eingangsanschlüssen (319) mit dem Rückkopplungseingangsanschluss derart verbunden ist, dass das ADC-Subsystem (318) das Rückkopplungssignal von der H-Brückenschaltung (334) empfängt, und wobei das ADC-Subsystem (318) konfiguriert ist, um analoge Signale, die an der Vielzahl von ADC-Eingangsanschlüssen (319) empfangen werden, mit einer Abtastfrequenz abzutasten, die proportional zu der Frequenz des Haupttaktsignals ist, und das ADC-Subsystem (318) konfiguriert ist, um unter Verwendung des abgetasteten analogen Signals digitale ADC-Signale zu erzeugen;
ein digitales Prozessorsubsystem, das konfiguriert ist, um die digitalen ADC-Signale von dem ADC-Subsystem (318) zu empfangen und die digitalen ADC-Signale zu verarbeiten, um das Treibersteuersignal zu erzeugen, wobei das digitale Prozessorsubsystem konfiguriert ist, um das Treibersteuersignal an das PWM-Signalgeneratorsubsystem zu übermitteln, um das PWM-Signalgeneratorsubsystem (329) zu steuern; und
ein Digital-Analog-Umsetzersubsystem (DAC-Subsystem), umfassend:
einen Digital-Analog-Umsetzer (DAC) (327), der konfiguriert ist, um ein digitales Steuersignal, das durch das digitale Prozessorsubsystem erzeugt wird, in ein analoges Spannungssteuersignal umzusetzen, um eine Spannungskonstanthalterschaltung zu steuern, die konfiguriert ist, um eine Spannung für eine Modulation durch die H-Brückenschaltung (334) zu erzeugen; und
einen Wechselstromausgangsanschluss, der konfiguriert ist, um das analoge Spannungssteuersignal auszugeben, um die Spannungskonstanthalterschaltung zu steuern, um eine vorbestimmte Spannung für die Modulation durch die H-Brückenschaltung (334) zu erzeugen, um einen Resonanzkreis als Reaktion auf Rückkopplungssignale anzutreiben, die den Betrieb des Resonanzkreises angeben.

2. Einrichtung nach Anspruch 1, wobei der Oszillator (315) konfiguriert ist, um das Haupttaktsignal mit einer Frequenz von 50 kHz bis 105 MHz zu erzeugen.

3. Einrichtung nach Anspruch 1 oder 2, wobei der Mikrochip ferner umfasst:
einen Frequenzteiler, der mit dem Oszillator (315) verbunden ist, um das Haupttaktsignal von dem Oszillator (315) zu empfangen, wobei der Frequenzteiler konfiguriert ist, um das Haupttaktsignal durch einen vorbestimmten Teilerbetrag zu teilen und das Frequenzreferenzsignal an die Verzögerungsregelschleife (332) auszugeben.

4. Einrichtung nach einem der vorstehenden Ansprüche, wobei die Verzögerungsregelschleife (332) eine Vielzahl von Verzögerungsleitungen umfasst, die in Reihe geschaltet sind, wobei die Gesamtverzögerung der Verzögerungsleitungen gleich der Periode des Haupttaktsignals ist.

5. Einrichtung nach Anspruch 4, wobei die Verzögerungsregelschleife (332) konfiguriert ist, um das Tastverhältnis des ersten Phasentaktsignals und des zweiten Phasentaktsignals als Reaktion auf das Treibersteuersignal durch Variieren der Verzögerung jeder Verzögerungsleitung in der Verzögerungsregelschleife (332) anzupassen.

6. Einrichtung nach einem der vorstehenden Ansprüche, wobei der Rückkopplungseingangsanschluss konfiguriert ist, um ein Rückkopplungssignal von der H-Brückenschaltung (334) in Form einer Spannung zu empfangen, die einen Effektivstrom des Wechselstromantriebssignals angibt, das den Resonanzkreis antreibt.

7. Einrichtung nach einem der vorstehenden Ansprüche, wobei das ADC-Subsystem (318) eine Vielzahl von weiteren ADC-Eingangsanschlüssen (319) umfasst, die konfiguriert sind, um Rückkopplungssignale zu empfangen, die mindestens eines von der Spannung einer Batterie, die mit der Einrichtung verbunden ist, oder der Spannung eines Batterieladegeräts angeben, das mit der Einrichtung verbunden ist.

8. Einrichtung nach einem der vorstehenden Ansprüche, wobei der Mikrochip ferner umfasst:
einen Temperatursensor, der innerhalb des Mikrochips eingebettet ist, wobei der Temperatursensor konfiguriert ist, um ein Temperatursignal zu erzeugen, das die Temperatur des Mikrochips angibt, und wobei das Temperatursignal durch einen weiteren ADC-Eingangsanschluss des ADC-Subsystems (318) empfangen wird und das Temperatursignal durch den ADC abgetastet wird.

9. Einrichtung nach einem der vorstehenden Ansprüche, wobei das ADC-Subsystem (318) konfiguriert ist, um Signale, die an der Vielzahl von ADC-Eingangsanschlüssen (319) empfangen werden, sequenziell abzutasten, wobei jedes Signal durch das ADC-Subsystem (318) eine jeweilige vorbestimmte Anzahl von Malen abgetastet wird.

10. Einrichtung nach einem der vorstehenden Ansprüche, wobei der Mikrochip ferner umfasst:
ein Batterieladesubsystem, das konfiguriert ist, um das Laden einer externen Batterie zu steuern, die mit dem Mikrochip verbunden ist.

11. Einrichtung nach einem der vorstehenden Ansprüche, wobei das DAC-Subsystem umfasst:
einen weiteren Digital-Analog-Umsetzer (DAC) (328), der konfiguriert ist, um ein weiteres digitales Steuersignal, das durch das digitale Prozessorsubsystem erzeugt wird, in ein weiteres analoges Spannungssteuersignal umzusetzen, um die Spannungskonstanthalterschaltung zu steuern.

## Revendications

1. Appareil de transmission d'ondes ultrasonores, l'appareil comprenant :
un circuit résonant, dans lequel le circuit résonant est au moins l'un parmi un circuit bouchon LC, une antenne et un transducteur piézoélectrique ;
un circuit en pont en H (334) connecté au circuit résonnant, dans lequel le circuit en pont en H (334) est configuré pour générer un signal de pilotage CA pour piloter le circuit résonnant afin de générer et de transmettre les ondes ultrasonores ;
une micropuce connectée au circuit de pont en H (334), dans lequel la micropuce est configurée pour commander le circuit de pont en H (334) pour générer le signal de pilotage CA, dans lequel la micropuce est une unité unique qui comprend une pluralité de composants et sous-systèmes intégrés interconnectés comprenant :
un oscillateur (315) qui est configuré pour générer :
un signal d'horloge principal,
un signal d'horloge de première phase qui est haut pendant un premier temps durant la demi-période positive du signal d'horloge principal et bas durant la demi-période négative du signal d'horloge principal, et
un signal d'horloge de seconde phase qui est haut pendant un second temps durant la demi-période négative du signal d'horloge principal et bas durant la demi-période positive du signal d'horloge principal, dans lequel les phases du signal d'horloge de première phase et du signal d'horloge de seconde phase sont alignées au centre ;
un sous-système de générateur de signal de modulation de largeur d'impulsion (PWM) (329) comprenant :
une boucle à verrouillage de retard (332) qui est configurée pour générer un signal d'horloge de double fréquence à l'aide du signal d'horloge de première phase et du signal d'horloge de seconde phase, le signal d'horloge de double fréquence étant le double de la fréquence du signal d'horloge principal, dans lequel la boucle à verrouillage de retard (332) est configurée pour commander le front montant du signal d'horloge de première phase et du signal d'horloge de seconde phase pour qu'il soit synchrone avec le front montant du signal d'horloge de double fréquence, et dans lequel la boucle à verrouillage de retard (332) est configurée pour ajuster la fréquence et le cycle de service du signal d'horloge de première phase et du signal d'horloge de seconde phase en réponse à un signal de commande de pilotage pour produire un signal de sortie de première phase et un signal de sortie de seconde phase, dans lequel le signal de sortie de première phase et le signal de sortie de seconde phase sont configurés pour piloter le circuit de pont en H (334) pour générer un signal de pilotage CA pour piloter le circuit résonant ;
une borne de signal de sortie de première phase qui est configurée pour délivrer en sortie le signal de sortie de première phase au circuit de pont en H (334) ;
une borne de signal de sortie de seconde phase qui est configurée pour délivrer en sortie le signal de sortie de seconde phase au circuit de pont en H (334) ;
une borne d'entrée de rétroaction qui est configurée pour recevoir un signal de rétroaction en provenance du circuit de pont en H (334), le signal de rétroaction indiquant un paramètre du fonctionnement du circuit de pont en H (334) ou du signal de pilotage CA lorsque le circuit de pont en H (334) est en train de piloter le circuit résonant avec le signal de pilotage CA ;
un sous-système de convertisseur analogique vers numérique (ADC) (318) comprenant :
une pluralité de bornes d'entrée d'ADC (319) qui sont configurées pour recevoir une pluralité de signaux analogiques respectifs, dans lequel une borne d'entrée d'ADC de la pluralité de bornes d'entrée d'ADC (319) est connectée à la borne d'entrée de rétroaction de telle sorte que le sous-système d'ADC (318) reçoit le signal de rétroaction en provenance du circuit de pont en H (334), et dans lequel le sous-système d'ADC (318) est configuré pour échantillonner des signaux analogiques reçus au niveau de la pluralité de bornes d'entrée d'ADC (319) à une fréquence d'échantillonnage qui est proportionnelle à la fréquence du signal d'horloge principal et le sous-système d'ADC (318) est configuré pour générer des signaux numériques d'ADC à l'aide des signaux analogiques échantillonnés ;
un sous-système de processeur numérique qui est configuré pour recevoir les signaux numériques d'ADC en provenance du sous-système d'ADC (318) et traiter les signaux numériques d'ADC pour générer le signal de commande de pilotage, dans lequel le sous-système de processeur numérique est configuré pour communiquer le signal de commande de pilotage au sous-système de générateur de signal PWM pour commander le sous-système de générateur de signal PWM (329) ; et
un sous-système de convertisseur numérique vers analogique (DAC) comprenant :
un convertisseur numérique vers analogique (DAC) (327) qui est configuré pour convertir un signal de commande numérique généré par le sous-système de processeur numérique en un signal de commande de tension analogique pour commander un circuit régulateur de tension qui est configuré pour générer une tension pour modulation par le circuit de pont en H (334) ; et
une borne de sortie de DAC qui est configurée pour délivrer en sortie le signal de commande de tension analogique pour commander le circuit régulateur de tension pour générer une tension prédéterminée pour modulation par le circuit de pont en H (334) pour piloter le circuit résonant en réponse à des signaux de rétroaction qui indiquent le fonctionnement du circuit résonant.

2. Appareil selon la revendication 1, dans lequel l'oscillateur (315) est configuré pour générer le signal d'horloge principal à une fréquence de 50 kHz à 105 MHz.

3. Appareil selon la revendication 1 ou la revendication 2, dans lequel la micropuce comprend en outre :
un diviseur de fréquence qui est connecté à l'oscillateur (315) pour recevoir le signal d'horloge principal en provenance de l'oscillateur (315), le diviseur de fréquence étant configuré pour diviser le signal d'horloge principal par une quantité de division prédéterminée et délivrer en sortie le signal de référence de fréquence à la boucle à verrouillage de retard (332).

4. Appareil selon l'une quelconque des revendications précédentes, dans lequel la boucle à verrouillage de retard (332) comprend une pluralité de lignes à retard connectées bout à bout, dans lequel le retard total des lignes à retard est égal à la période du signal d'horloge principal.

5. Appareil selon la revendication 4, dans lequel la boucle à verrouillage de retard (332) est configurée pour ajuster le cycle de service du signal d'horloge de première phase et du signal d'horloge de seconde phase en réponse au signal de commande de pilotage en faisant varier le retard de chaque ligne à retard dans la boucle à verrouillage de retard (332).

6. Appareil selon l'une quelconque des revendications précédentes, dans lequel la borne d'entrée de rétroaction est configurée pour recevoir un signal de rétroaction en provenance du circuit de pont en H (334) sous la forme d'une tension qui indique un courant efficace du signal de pilotage CA qui est en train de piloter le circuit résonant.

7. Appareil selon l'une quelconque des revendications précédentes, dans lequel le sous-système d'ADC (318) comprend une pluralité de bornes d'entrée d'ADC supplémentaires (319) qui sont configurées pour recevoir des signaux de rétroaction qui indiquent au moins l'une parmi la tension d'une batterie connectée à l'appareil ou la tension d'un chargeur de batterie connecté à l'appareil.

8. Appareil selon l'une quelconque des revendications précédentes, dans lequel la micropuce comprend en outre :
un capteur de température qui est intégré au sein de la micropuce, dans lequel le capteur de température est configuré pour générer un signal de température qui indique la température de la micropuce, et dans lequel le signal de température est reçu par une borne d'entrée d'ADC supplémentaire du sous-système d'ADC (318) et le signal de température est échantillonné par l'ADC.

9. Appareil selon l'une quelconque des revendications précédentes, dans lequel le sous-système d'ADC (318) est configuré pour échantillonner des signaux reçus au niveau de la pluralité de bornes d'entrée d'ADC (319) séquentiellement, chaque signal étant échantillonné par le sous-système d'ADC (318) un nombre de fois prédéterminé respectif.

10. Appareil selon l'une quelconque des revendications précédentes, dans lequel la micropuce comprend en outre :
un sous-système de charge de batterie qui est configuré pour commander la charge d'une batterie externe qui est connectée à la micropuce.

11. Appareil selon l'une quelconque des revendications précédentes, dans lequel le sous-système de DAC comprend :
un convertisseur numérique vers analogique (DAC) supplémentaire (328) qui est configuré pour convertir un signal de commande numérique supplémentaire généré par le sous-système de processeur numérique en un signal de commande de tension analogique supplémentaire pour commander le circuit régulateur de tension.
